# EUROPEAN PATENT SPECIFICATION

(11) **EP 2 729 485 B1**
(45) Date of publication and mention of the grant of the patent: **07.06.2017**
(21) Application number: 12735458.7
(22) Date of filing: 09.07.2012
(51) Int. Cl.: C07K 5/083, C07K 5/10, A61K 38/06, A61K 38/07, A61K 38/08, C07K 7/06, A61P 25/16, A61P 25/28, A61P 3/10, C07K 5/093, C07K 5/103, C07K 5/09

(54) **ANTI-AMYLOIDOGENIC, ALPHA-HELIX BREAKING ULTRA-SMALL PEPTIDE THERAPEUTICS**
ANTI-AMYLOIDOGENE ALPHA-HELIX-BRECHENDE ULTRAKLEINE PEPTIDTHERAPEUTIKA
PRODUITS THÉRAPEUTIQUES PEPTIDIQUES ULTRA PETITS DE RUPTURE D'HÉLICE ALPHA, ANTI-AMYLOÏDOGÈNES

(30) Priority: 07.07.2011 SG 201104978
(43) Date of publication of application: 14.05.2014
(73) Proprietor: Agency for Science, Technology and Research, Singapore 138632 (SG)
(72) Inventor: HAUSER, Charlotte, Singapore 138669 (SG); LAKSHMANAN, Anupama, Singapore 138669 (SG)
(74) Representative: Engelhard, Markus
(86) International application number: PCT/EP2012/002890
(87) International publication number: WO 2013/004399

(56) References cited:
- WO-A1-95/05841
- WO-A2-01/34631
- WO-A2-2006/127702
- US-B1- 7 632 819
- MASMAN M F ET AL: "Synthesis and conformational analysis of His-Phe-Arg-Trp-NH2 and analogues with antifungal properties", BIOORGANIC & MEDICINAL CHEMISTRY, PERGAMON, GB, vol. 14, no. 22, 15 November 2006 (2006-11-15), pages 7604-7614, XP027992192, ISSN: 0968-0896 [retrieved on 2006-11-15]
- BORCHARDT A ET AL: "SYNTHETIC RECEPTOR BINDING ELUCIDATED WITH AN ENCODED COMBINATORIAL LIBRARY", JOURNAL OF THE AMERICAN CHEMICAL SOCIETY, ACS PUBLICATIONS, US, vol. 116, no. 1, 12 January 1994 (1994-01-12), page 373/374, XP002063107, ISSN: 0002-7863, DOI: 10.1021/JA00080A044
- TONG XU, R. MARSHALL WERNER, KWUN-CHI LEE, JAMES C. FETTINGER, JEFFERY T. DAVIS, JAMES K. COWARD: "Synthesis and Evaluation of Tripeptides Containing Asparagine Analogues as Potential Substrates or Inhibitors of Oligosaccharyltransferase", JOURNAL OF ORGANIC CHEMISTRY, vol. 63, 23 June 1998 (1998-06-23), pages 4767-4778, XP002682494,
- GIUSEPPE MELACINI, YANGBO FENG, MURRAY GOODMAN: "Collagen-Based Structures Containing the Peptoid Residue N-Isobutylglycine (Nleu). 6. conformational Analysis of Gly-Pro-Nleu Sequences by 1H NMR, CD, and Molecular Modeling", JOURNAL OF THE AMERICAN CHEMICAL SOCIETY, vol. 118, no. 44, 6 November 1996 (1996-11-06), pages 10725-10732, XP002682495,
- COCINERO EMILIO J ET AL: "Peptide Secondary Structures in the Gas Phase: Consensus Motif of N-Linked Glycoproteins", JOURNAL OF THE AMERICAN CHEMICAL SOCIETY, ACS PUBLICATIONS, US, vol. 131, no. 3, 28 January 2009 (2009-01-28), pages 1282-1287, XP008150743, ISSN: 0002-7863, DOI: 10.1021/JA808687J [retrieved on 2008-12-30]
- MARTIJN VERDOES, LIANNE I. WILLEMS, WOUTER A. VAN DER LINDEN, BOUDEWIJN A. DUIVENVOORDEN, GIJSBERT A. VAN DER MAREL ET AL: "A panel of subunit-selective activity-based proteasome probes", ORGANIC & BIOMOLECULAR CHEMISTRY, vol. 8, 6 May 2010 (2010-05-06), pages 2719-2727, XP002682496,
- ROBERT C. THOMPSON, ELKAN R. BLOUT: "Dependence of the Kinetic Parameters for Elastase-Catalyzed Amide Hydrolysis on the Length of Peptide Substrates", BIOCHEMISTRY, vol. 12, no. 1, January 1973 (1973-01), pages 57-65, XP002682497,
- DIETER BRÖMME AND ROLF KLEINE: "Substrate Specificity of Thermitase, a Thermostable Serine Proteinase from Thermoactinomyces vulgaris", CURRENT MICROBIOLOGY, vol. 11, no. 2, 1984, pages 93-99, XP008155495,
- VOLKER SCHELLENBERGER, KATRIN BRAUNE, HANS-JÖRG HOFMANN, HANS-DIETER JAKUBKE: "The specificity of chymotrypsin: A statistical analysis of hydrolysis data", EUROPEAN JOURNAL OF BIOCHEMISTRY, vol. 199, no. 3, August 1991 (1991-08), pages 623-636, XP002682498,

## Description

The invention provides ultra-small peptide inhibitors that are capable of preventing amyloid formation/amyloidosis.

The inventors have recently found that a class of systematically designed ultra-small peptides is able to form amyloid structures by a stepwise formation. The initiation step occurs via crucial α-helical intermediate structures that are established before the final β-type amyloid structure is formed. The rationale for the development of anti-amyloidogenic peptide therapeutics is based on the idea of using inhibitory peptides that prevent the formation of α-helical intermediate structures.

Amyloids are tissue deposits of insoluble, proteinaceous fibrils that are rich in cross β-pleated sheet structure. The process of amyloid fibril formation is a key event in diverse and structurally unrelated pathological processes. These include many chronic, debilitating and increasingly prevalent diseases that can be broadly classified as: 1) neuro-degenerative, e.g. Alzheimer's, Parkinson's, Huntington's, 2) non-neuropathic localized amyloidosis such as in Type II Diabetes, and 3) systemic amyloidosis that occurs in multiple tissues. Despite differences in symptoms and protein monomers associated with these protein misfolding disorders (PMDs), there seems to be a common mechanism underlying protein aggregation at the molecular level. The formation of amyloid fibrils is through a molecular recognition and self-assembly process that typically starts with a thermodynamically unfavourable lag phase for the formation of a 'nucleus or seed'. This is followed by a thermodynamically favourable exponential growth phase, where monomers/oligomers are added to the growing nucleus. The conformational transition of the protein from a random-coiled soluble form via an α-helical intermediate into insoluble, cross β-pleated fiber aggregates is thought to be a key event in amyloidogenesis.

While recent scientific research has focussed on gaining more insight into the mechanism of molecular recognition and self-assembly in amyloidosis for inhibiting this process, there are still no effective preventions or treatments for any of these diseases. Small molecules that safely antagonize and prevent amyloidogenesis are desperately needed as therapeutics. The anti-amyloidogenic candidates should also be able to fulfil stringent requirements, such as efficient and easy uptake, sufficient half-life and circulation time in vivo, non-toxicity, and permeability through the blood-brain barrier (BBB), which is needed for treating neuro-degenerative conditions. Furthermore, since there is increasing evidence that pre-fibril oligomer intermediates may be even more toxic than mature amyloid fibers, it is important to arrest or reverse the self-assembly process at an early stage.

Thus, it was an object of the present invention to provide novel compounds that have the above properties.

The objects are solved according to the invention as it is claimed in the claims.

The objects of the present invention are solved by an isolated alpha-helix breaking peptide for use in the treatment of a disease associated with amyloidosis, said isolated alpha-helix breaking peptide having the general formula

Z-(X)ₘ-Proline-(X)ₙ,

wherein
Z is an N-terminal protecting group;
X is, at each occurrence, independently selected from non-aromatic amino acids; and m and n indicate the number of amino acids and amino acid derivatives and are integers independently selected from 1 to 5, with m + n being ≤ 6,
wherein said N-terminal protecting group has the general formula -C(O)-R, R being selected from the group consisting of methyl, ethyl, propyl, isopropyl, butyl and isobutyl, and
wherein the N-terminal amino acid of said peptide is more hydrophobic than the C-terminal amino acid of said peptide.

Proline is abbreviated using either the three-letter code (Pro) or one-letter code (P).

In one embodiment, m + n is ≤ 5, preferably ≤ 4, more preferably ≤ 3.

"Amino acids" include naturally occurring L- and D-amino acids.

In one embodiment, X is, at each occurrence, independently selected from naturally occurring amino acids.

In one embodiment, at least one X is a D-amino acid.

In one embodiment, said non-aromatic amino acids are selected from the group consisting of isoleucine (Ile, I), leucine (Leu, L), valine (Val, V), alanine (Ala, A), glycine (Gly, G), aspartic acid (Asp, D), asparagine (Asn, N), glutamic acid (Glu, E), glutamine (Gln, Q), serine (Ser, S), threonine (Thr, T) and lysine (Lys, K).

According to the invention, the N-terminal amino acid of said peptide is more hydrophobic than the C-terminal amino acid of said peptide. In one embodiment, the hydrophobicity decreases from the N-terminus to the C-terminus.

In one embodiment, m and n are 1, i.e. said peptide has the general formula

Z-X-Proline-X.

According to the invention, said N-terminal protecting group has the general formula -C(O)-R, wherein R is selected from the group consisting of methyl, ethyl, propyl, isopropyl, butyl and isobutyl.

In a preferred embodiment, said N-terminal protecting group is an acetyl group (R = methyl).

In one embodiment, the C-terminus of said peptide is amidated or esterified, wherein, preferably, the C-terminus has the formula -CONHR, with R being selected from the group consisting of H, alkyl and substituted alkyl, or the formula -COOR, with R being selected from the group consisting of alkyl and substituted alkyl.

In one embodiment, said peptide is provided in an aqueous solution, optionally comprising a physiological buffer.

In one embodiment, said peptide is based on a peptide selected from the group consisting of *Z*-LIVAGDD (SEQ ID NO: 1), *Z*-LIVAGEE (SEQ ID NO: 2), *Z*-LIVAGD (SEQ ID NO: 3), *Z*-ILVAGD (SEQ ID NO: 4), *Z*-LIVAAD (SEQ ID NO: 5), *Z*-LAVAGD (SEQ ID NO: 6), *Z-*AIVAGD (SEQ ID NO: 7), *Z*-LIVAGE (SEQ ID NO: 8), *Z*-LIVAGK (SEQ ID NO: 9), *Z-*LIVAGS (SEQ ID NO: 10), *Z*-ILVAGS (SEQ ID NO: 11), *Z*-AIVAGS (SEQ ID NO: 12), *Z-*LIVAGT (SEQ ID NO: 13), *Z*-AIVAGT (SEQ ID NO: 14), *Z*-LIVAD (SEQ ID NO: 15), *Z-*LIVGD (SEQ ID NO: 16), *Z*-IVAD (SEQ ID NO: 17), *Z*-IIID (SEQ ID NO: 18), *Z*-IIIK (SEQ ID NO: 19) and Z-IVD (SEQ ID NO: 20), wherein one amino acid except the N-terminal and C-terminal amino acid is replaced with a Proline (Pro, P). For example, the isolated alpha-helix breaking peptide according to the present invention may be based on the peptide *Z-*LIVAGD (SEQ ID NO: 3) of the above list, and thus be selected from *Z*-LPVAGD (SEQ ID NO: 21), *Z*-LIPAGD (SEQ ID NO: 22), *Z*-LIVPGD (SEQ ID NO: 23) and *Z*-LIVAPD (SEQ ID NO: 24).

In one embodiment, said peptide is selected from the group consisting of from *Z*-LPVAGD (SEQ ID NO: 21), *Z*-LIPAGD (SEQ ID NO: 22), Z-LIVPGD (SEQ ID NO: 23), *Z*-LIVAPD (SEQ ID NO: 24), *Z*-IPD (SEQ ID NO: 25), *Z*-NPI (SEQ ID NO: 26), *Z*-IPN (SEQ ID NO: 27), *Z*-IPI (SEQ ID NO: 28), *Z*-APA (SEQ ID NO: 29), *Z*-LPI (SEQ ID NO: 30), *Z*-IPL (SEQ ID NO: 31), *Z*-LPL (SEQ ID NO: 32), *Z*-APF (SEQ ID NO: 33), *Z*-KPA-CONH₂ (SEQ ID NO: 34), *Z*-LPD (SEQ ID NO: 35), *Z*-LPE (SEQ ID NO: 36), *Z*-IPK-CONH₂ (SEQ ID NO: 37), *Z*-APD (SEQ ID NO: 38), *Z*-IPF (SEQ ID NO: 39), *Z*-IPS (SEQ ID NO: 40), *Z*-IPW (SEQ ID NO: 41), *Z*-APS (SEQ ID NO: 42), *Z*-NPK-CONH₂ (SEQ ID NO: 43) and *Z*-LPG (SEQ ID NO: 44). SEQ ID NOs: 45 to 60 represent particular embodiments of the above sequences.

As discussed above, the objects of the present invention are solved by an isolated alpha-helix breaking peptide as defined above for use in the treatment of a disease associated with amyloidosis.

In one embodiment, said disease associated with amyloidosis is selected from the group consisting of Neuro-degenerative diseases, such as Alzheimer's disease, Parkinson's disease, Huntington's disease, Amyotrophic lateral sclerosis (ALS) and Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia 17, Spinal and bulbar muscular atrophy, Hereditary dentatorubral-pallidoluysian atrophy, Familial British dementia, Familial Danish dementia, Non-neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAI amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis.
In one embodiment, said peptide is administered orally.
The objects of the present invention are also solved by a pharmaceutical composition comprising an isolated alpha-helix breaking peptide as defined above.
In one embodiment, said pharmaceutical composition further comprises at least one pharmaceutically acceptable carrier, diluent and/or excipient.
In one embodiment, said pharmaceutical composition further comprises a monovalent or divalent metal salt, preferably a divalent metal salt.
In one embodiment, said metal is selected from the group consisting of sodium, magnesium, calcium and zinc.

The present disclosure describes a method of treatment of a disease associated with amyloidosis, said method comprising the step of administering an isolated alpha-helix breaking peptide as defined above or a pharmaceutical composition as defined above to a person in need thereof. For example, said isolated alpha-helix breaking peptide or said pharmaceutical composition are administered orally.

The present disclosure further describes a method of disaggregating an amyloid plaque or preventing formation thereof, said method comprising contacting an isolated alpha-helix breaking peptide as defined above with said amyloid plaque, thereby disaggregating said amyloid plaque or preventing formation thereof.

The method can further comprise contacting said amyloid plaque with a monovalent or divalent metal salt, such as a divalent metal salt, wherein said metal can be sodium, magnesium, calcium or zinc.

The inventors have surprisingly found that specific rationally designed ultra-small peptides can inhibit amyloidogenesis. These inhibitor peptides consist of 3-7 natural amino acids that are capable of interfering with and preventing α-helical intermediate structures. These intermediate structures are thought to be important conformational transition states that drive the formation of amyloid aggregates, hence directing amyloidogenesis. Specifically, the inventors believe that the conformational change of the protein from random coiled to α-helix plays an important part in molecular self-recognition. By breaking or inhibiting the transition to α-helix, one can stop/reverse the amyloid aggregation process at a very early stage (i.e. immediately or within a few minutes). Thus, these anti-amyloidogenic peptides can also be considered as α-helical breakers. They are capable of recognizing and interacting with the short, amyloidogenic recognition motifs of misfolded proteins, while themselves having a very poor propensity to self-assemble into ordered supramolecular structures. By virtue of their short length, especially for the 3-mers, they have the potential to evade protease recognition and degradation, while facilitating easy oral delivery and BBB permeability. In addition, these ultra-small peptides are non-toxic, since they are made of non-toxic amino acids and amino acid derivatives, preferably naturally occurring amino acids. Furthermore, in order to assist disaggregation of already existing amyloids, the presence of mono- or divalent metal salts is preferred.

In summary, the small size of these ultra-short peptides enables easy and effective oral uptake, which is a key advantage in drug delivery. Furthermore, the small size of the ultra-short peptides guarantees simple batch synthesis at low costs. Because, in a preferred embodiment, the peptide-based therapeutics are made of naturally occurring amino acids they are non-toxic, non-immunogenic and biocompatible. Ultra-short peptides that are just three amino acids in length are also able to cross the blood-brain barrier, which is a key requirement for therapeutics targeted against neuro-degenerative disorders. Finally, the short peptide motif is likely to evade recognition and degradation by endogenous peptidases, hence providing greater half-life and bio-availability in biological fluids and tissues.

Development of an effective, small molecule drug that inhibits amyloidogenesis has huge and attractive market potential, especially since there is extensive evidence indicating the common molecular basis of amyloid aggregation in widespread diseases. For instance, Alzheimer's, just one of the many diseases involving amyloidosis, is the most frequent cause of late-life dementia (50-70%) and a leading cause of death in the developed world. In 2010, there was an estimated 35.6 million people with dementia, with the number expected to almost double to 65.7 million by 2030 and reach 115.4 million by 2050. Another example is Type II diabetes, which accounts for 90-95% of all diagnosed cases of diabetes in adults. As currently available oral drugs are targeted at maintaining good control of blood glucose, there is a need to develop oral therapeutics that prevent formation of islet amyloid that is responsible for pancreatic cell death. Several of the current drugs also have serious side-effects that will be overcome by the use of natural occurring biological macromolecules. In this regard, the ultra-small inhibitor peptides according to the present invention have promising potential to address current therapeutic needs, while simultaneously trying to overcome the problems and limitations of existing drugs.

### Reference is now made to the figures, wherein

**Figure 1** shows circular dichroism (CD) spectra of the peptides Ac-LD₆, Ac-NL₆ and Ac-ID₃ in solution (at lower concentrations) and in hydrogel form (at higher concentrations);
**Figure 2** shows Thioflavin T (ThT) binding to Alzheimer's core sequence KE₇ correlating with an increase in fluorescence over time (= positive control; 100µM). Nine independent samples were measured at each time point (gain value set for the experiment: 55);
**Figures 3** to **17** show Thioflavin T (ThT) binding to Alzheimer's core sequence KE₇ in absence and presence of inhibitor peptides 1 to 5 and 7 to 16. Nine independent samples were measured at each time point (gain value set for the experiment: 55);
**Figure 18** shows the distribution of control values (water and ThT dye);
**Figures 19** and **20** show that the inhibitor peptides themselves do not give enhanced ThT fluorescence;
**Figure 21** summarizes the screening procedure for inhibitor peptides of the present invention;
**Figure 22** shows morphological studies of inhibitor peptides of the present invention using field emission scanning electron microscopy (FESEM);
**Figure 23** shows a hemolysis assay testing the biocompatibility of inhibitor peptides of the present invention;
**Figure 24** shows the storage modulus G' / mechanical strength of hydrogels derived from 10 mg/mL of Ac-LD₆ (L) as a function of angular frequency (rad/sec) at different NaCl concentrations;
**Figure 25** shows a circular dichroism (CD) spectrum of the peptide Ac-NL₆ at three different concentrations (A) and hydrogels of the peptides Ac-NL₆ and Ac-LD₆ (B);
**Figure 26** shows rheological data for hydrogels formed by 1.5mM of Ac-KE₇ (green) and peptide-inhibitor solutions (no gelation) when 1.5mM of Ac-KE₇ was mixed with Ac-LPE (wine red) and Ac-LPG (red) in a 1:20 molar ratio. The graphs display the storage moduli (G' in Pa) as a function of (A) angular frequency (rad/sec) under 1% strain and (B) oscillation strain (%) at an angular frequency of 1 rad/sec at room temperature of 25°C;
**Figure 27** shows the results of a live/dead cytotoxicity assay using U87 human glioblastoma cells for the inhibitor peptides Ac-LG₃ and Ac-LE₃ at different concentrations and with or without neutralization of the inhibitor solution;
**Figure 28** shows the results of a WST-1 assay for the inhibitor peptides Ac-LG₃ and Ac-LE₃ at different concentrations and with or without neutralization of the inhibitor solution;
**Figure 29** shows the results of a live/dead cytotoxicity assay using HeLa and SHSY5Y neuroblastoma cells for an inhibitor peptide of the present invention at different concentrations and with or without neutralization of the inhibitor solution. Values expressed in mg/ml refer to the final concentration of the inhibitor in each well of a 96-well plate;
**Figure 30** shows the IC₅₀ sigmoidal curves of the inhibitor peptides Ac-LPG and Ac-LPE;
**Figure 31** shows a scheme of the solid-phase peptide synthesis used for the production of the inhibitor peptides of the present invention and an example of their characterization by ¹H NMR and LC-MS; and
**Figure 32** shows the results of a gelation study of the peptides Ac-KE₇ and Ac-NL₆ in absence and presence of inhibitor peptides 1 and 2 **(A)** and FESEM images of an inhibitor according to the present invention **(B).**

The present invention is now further described by means of the following examples, which are meant to illustrate the present invention.

### Experimental Procedures

Peptide-based hydrogel preparation. All peptides (purity ≥ 95%) were purchased from the American Peptide Company, Sunnyvale; following stringent quality control measures and amino acid analysis. All the peptides were acetylated at the N-terminus to suppress the effect of end charges. The peptides were dissolved in hot milliQ water (60-70°C) by vortexing for 5 minutes and left undisturbed at room temperature to form hydrogels. Depending on the peptide concentration, the self-assembly process occurred immediately, within hours or even within days (experimental time frame for gelation).

Circular dichroism (CD) studies. CD spectra were collected with an Aviv 410 CD spectrophotometer fitted with a Peltier temperature controller, using a rectangular quartz cuvette with a fitted cap and an optical path length optimal for the concentration of the peptide sample (e.g. 1 mm). For higher peptide concentration (10-20 mg/ml), quartz cuvettes with optical path lengths of 0.01 mm were used. Data acquisition was performed in steps of 0.5 nm or 1.0 nm at a wavelength range from 180-260 nm with a spectral bandwidth of 1.0 nm. To ensure reproducibility of the CD spectra, 3 samples of each peptide were individually measured, but the spectra were not averaged. All spectra were baseline-corrected with milliQ water as the reference.

Field emission scanning electron microscopy (FESEM) studies. Hydrogel samples were frozen at -20°C or better at -80°C. Frozen samples were then freeze-dried. Freeze-dried samples were fixed onto a sample holder using conductive tape and sputtered with platinum from both the top and the sides in a JEOL JFC-1600 High Resolution Sputter Coater. The coating current used was 30 mA and the process lasted for 60 sec. The surface of interest was then examined with a JEOL JSM-7400F Field Emission Scanning Electron Microscopy system using an accelerating voltage of 5-10 kV.

Rheology. To determine the viscoelastic properties/behaviour, peptide-based hydrogels were subjected to dynamic time, strain and frequency sweep experiments using the ARES-G2 rheometer (TA Instruments, Piscataway, NJ) with a 25.0 mm diameter stainless steel or titanium parallel plate geometry and a 0.8 mm or 1-2 mm gap distance. To ensure complete gelation and take account of the varying gelation speeds of different peptides at different concentrations, the rheology measurements were done 2 months after sample preparation. Oscillatory frequency sweep studies (measuring storage modulus (G') vs. Angular frequency (ω)) were done at 0.1-100 rad/s using 1% strain. Oscillatory amplitude sweep studies (measuring storage modulus (G') vs. oscillation strain % (y)) were done using 0.01-100% strain with a constant angular frequency of 1 rad/s. All measurements were carried out at 25 °C.

Biocompatibility: cell toxicity/viability assays. Biocompatibility of inhibitor peptide solutions with mammalian cells was evaluated qualitatively as well as quantitatively. For the qualitative assay, the live/dead cytotoxicity assay was employed to stain live and dead cells after incubation with the peptide solutions for 48-96 hours. Quantitative determination of cell viability was performed using the WST-1 reagent from Roche. Cell lines, such as U87 glioblastoma and SHSY5Y neuroblastoma, were specifically chosen as these are neuronal cell lines which are more relevant to drug candidates designed for treating Alzheimer's amyloid plaques. WST-1 is a stable tetrazolium salt that is cleaved into a soluble formazan (colored product) by a complex cellular mechanism. Since the reduction mainly depends on the glycolytic production of NAD(P)H in viable cells, the amount of formazan dye formed correlates directly to the number of metabolically active cells in the culture. For the quantitative assays, 5,000 cells/well for HeLa and U87 cells and 20,000 cells/well for SHSY5Y neuroblastoma cells were seeded in a 96-well plate. After incubation with the inhibitor peptides for 48 or 72 hours, the cell viability/cytotoxicity was evaluated. All the inhibitor solutions were prepared in plain growth medium without serum or other additives/growth factors. Neutralization of inhibitor solutions was done with 5M NaOH until pH reached the neutral range.

Biocompatibility: hemolysis assay. 1 ml of fresh rabbit blood was taken and washed 3 times in cold PBS (pH ∼7.3) solution. The final pelleted red blood cells (RBCs) were re-suspended in 4 ml of cold PBS and used for the assay. 1x PBS (pH∼ 7.3) was used as the negative control and 1% Triton-X in PBS was used as the positive control. 160 µl of the inhibitor peptide solution was mixed with 40 µl of the fresh RBC solution and incubated for 1 hour at 37°C. Five replicates were done for each sample. The samples were then centrifuged to allow the intact RBCs to settle down at the bottom and absorbance of 100 µl of the supernatant was measured at 567 nm using a plate reader.

Determination of IC₅₀ values. Solutions containing ThT, self-assembling peptides and inhibitors (total volume of 100 µL/well) were filled into the wells of a Greiner 96-well plate (GRE96ft), and fluorescence intensities were measured with a fluorescence plate reader (Tecan Safire 2) at one-minute intervals. The optimized measurement parameters are: excitation wavelength 452 nm; excitation bandwidth 9 nm; emission wavelength 485 nm; emission bandwidth 20 nm; gain value 45; temperature 26 to 28 °C. Varying molar excess of inhibitors was added to 100 µM of KE₇. The IC₅₀ sigmoidal curve was plotted from the results of five independent assays with at least 5 replicates in each assay. For the sigmoidal curve, the ThT fluorescence intensities detected at a particular time point (where the difference was maximum) were plotted against the log values of the concentrations of inhibitors.

Gelation studies. The natural core amyloidogenic sequences NL₆ (from Human Amylin) and KE₇ (from Alzheimer's Amyloid-Beta) were mixed with the inhibitor peptides. 1.5 mM of the core sequences (final concentration) was mixed with 10 and 20 Molar excess of the inhibitors. Milli-Q water at room temperature was used for dissolving the peptides, which were then left undisturbed for 3 months.

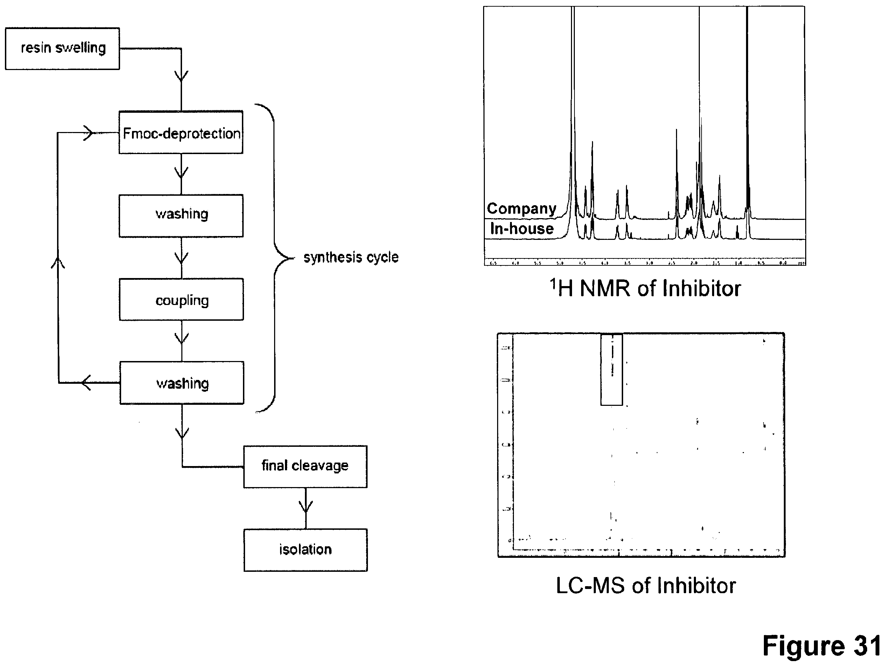

Inhibitors: synthesis and characterization. Solid-phase peptide synthesis was performed in accordance with Kirin et al. (2007) J. Chem. Educ. 84:108-111. All reactions were carried out in a handheld syringe. The synthesized products (starting material ∼ 1g of resin; gross weight of crude peptide ∼ 170 mg) were highly soluble in water and characterized by standard Mass Spectrometry (MS) and Nuclear Magnetic Resonance (¹H NMR in D₂O) techniques (see **Figure 31****).**

### Results

### CD spectra and rheology data of hydrogel-forming tri- and hexapeptides

Ac-LD₆ refers to the hexapeptide Ac-LIVAGD (see SEQ ID NO: 3), Ac-NL₆ refers to the hexapeptide Ac-NFGAIL (SEQ ID NO: 61), and Ac-ID₃ refers to the tripeptide Ac-IVD (see SEQ ID NO: 20). The peptide NFGAIL is a naturally occurring core sequence in human Amylin implicated in diabetes type 2, while the peptides LIVAGD and IVD are rationally designed peptides.

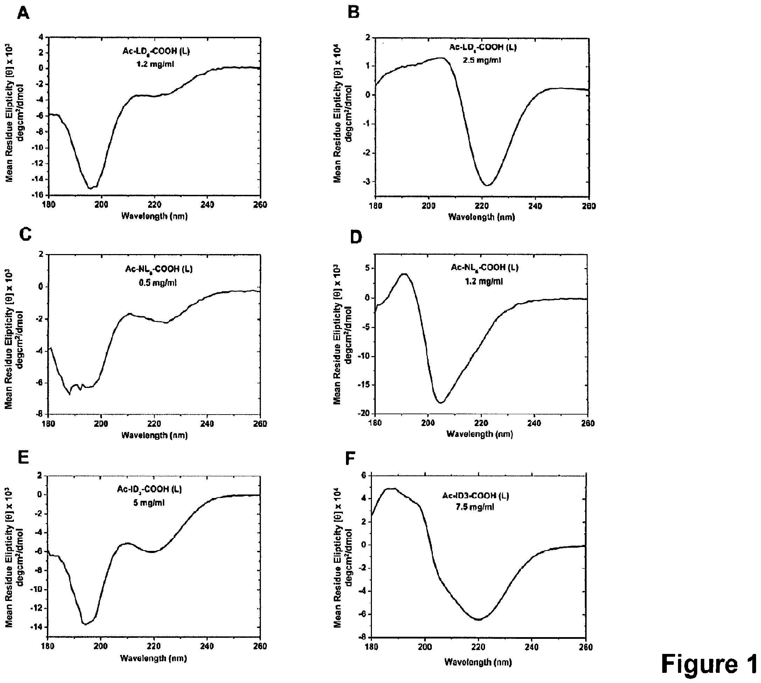

**Figure 1** shows CD spectra of 1.2 mg/ml Ac-LD₆ **(A),** 0.5 mg/ml Ac-NL₆ **(C)** and 5 mg/ml Ac-ID₃ **(E),** which demonstrate transition of the ultra-small peptides from random coil conformation to the meta-stable, transient α-helical state (minimum at 222 nm). At higher concentrations of 2.5 mg/ml Ac-LD₆ **(B),** 1.2 mg/ml Ac-NL₆ **(D)** and 7.5 mg/ml Ac-ID₃ **(F),** β-type structures are observed (minimum at around 220 nm; slightly blue shifted for the aromatic peptide sequence). All spectra were measured at room temperature of 25 °C. In **(F),** the equilibrium transition process from α-helical intermediates to β-type structures can be seen (shoulder at 208 nm).

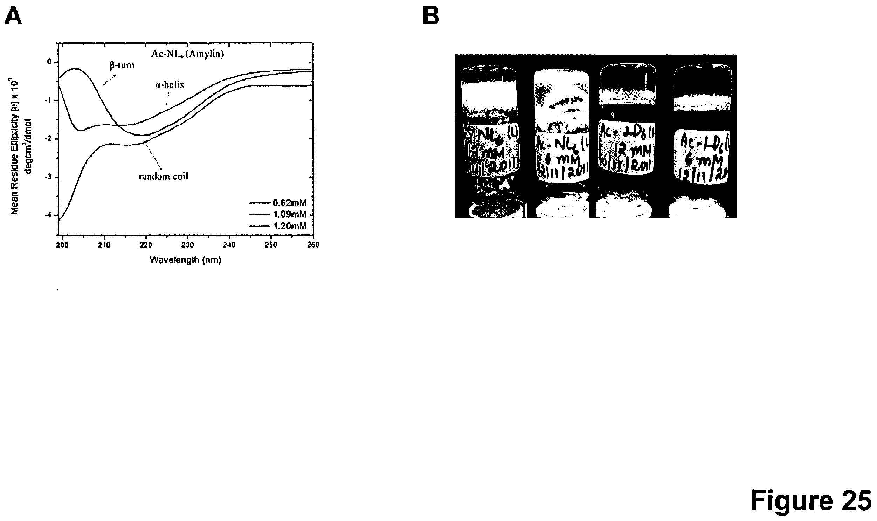

**Figure 25** **A** also shows that the natural core sequence NL₆ exhibits alpha-helical intermediates. Both the natural core sequence NL₆ and the designed peptide LD₆ form hydrogels at different concentrations (see **Figure 25 B)****.**

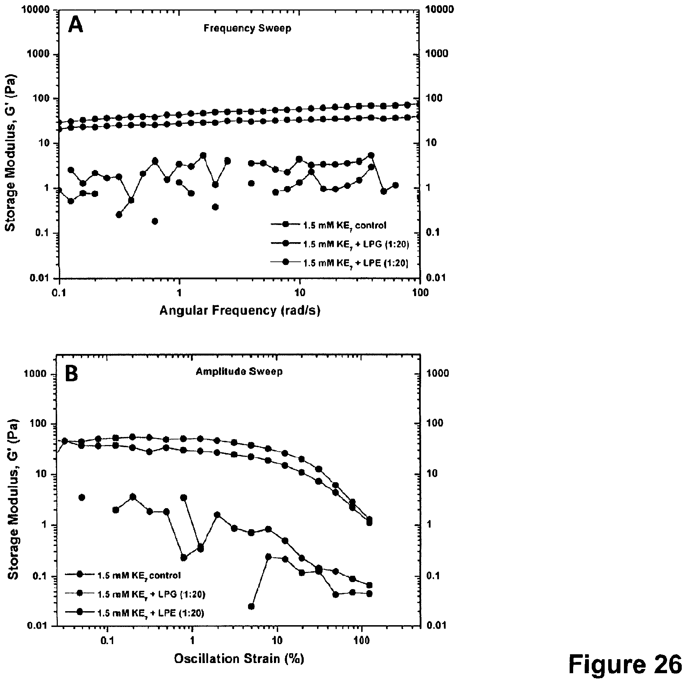

**Figure 26** shows rheological data for hydrogels formed by 1.5 mM of Ac-KE₇ (green) and peptide-inhibitor solutions (no gelation) when 1.5mM of Ac-KE₇ was mixed with Ac-LPE (wine red) and Ac-LPG (red) in a 1:20 molar ratio. The graphs display the storage moduli (G' in Pa) as a function of (A) angular frequency (rad/sec) under 1% strain and **(B)** oscillation strain (%) at an angular frequency of 1 rad/sec at room temperature of 25°C. The Ac-KE₇ controls without added inhibitors formed hydrogels that showed a linear viscoelastic range for the amplitude sweep and a linear profile for frequency sweep measurements; which were characteristic of this self-assembling peptide. However, the same concentration of Ac-KE₇ did not form a hydrogel when mixed with the inhibitor in a 1:20 molar ratio. This can be seen from the rheological data, where the peptide-inhibitor solutions give a highly fluctuating curve for both the amplitude and frequency sweep measurements because they are in solution form; instead of a gel form.

### Anti-amyloid peptide therapeutics ultra-small peptide inhibitors for oral uptake)

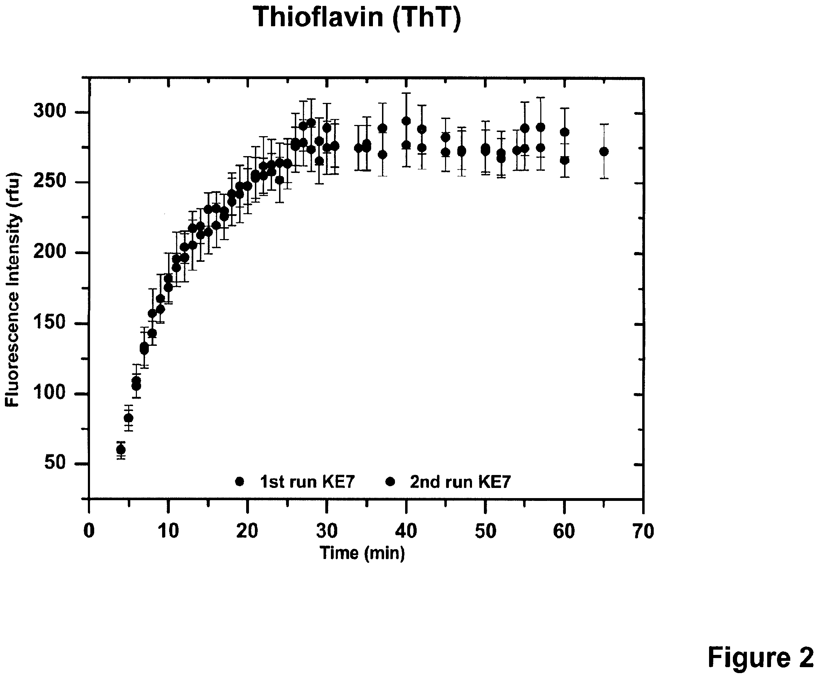

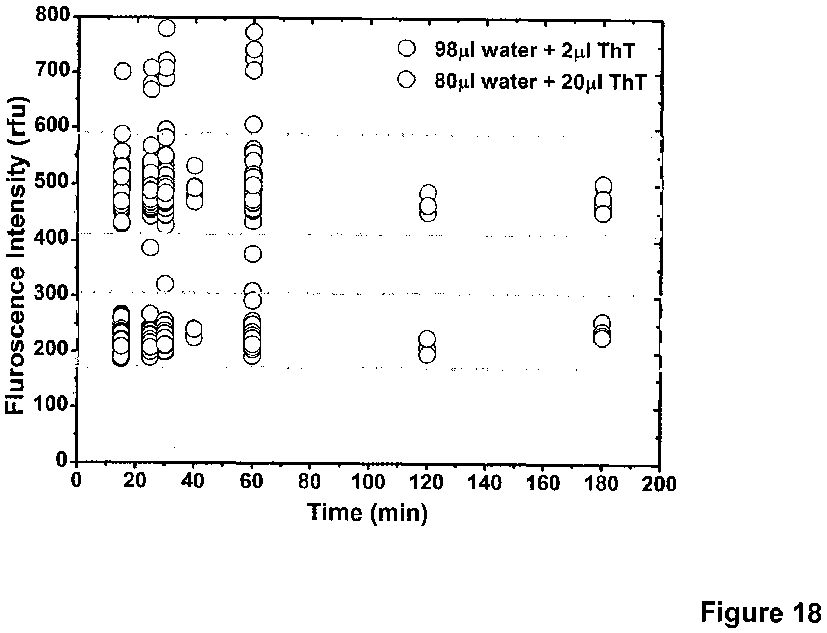

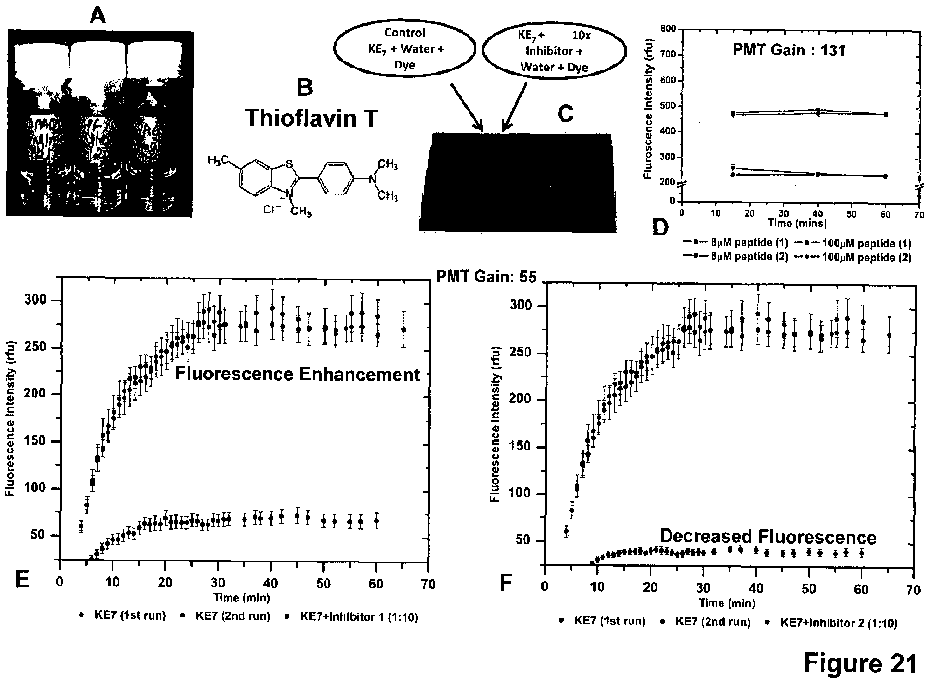

Thioflavin T (Basic Yellow 1 or CI 49005) is a benzothiazole salt obtained by the methylation of dehydrothiotoluidine with methanol in the presence of hydrochloric acid (see **Figure 21 B)****.** The dye is widely used to visualize and quantify the presence of misfolded protein aggregates called amyloid, both *in vitro* and *in vivo* (e.g., plaques composed of amyloid beta found in the brains of Alzheimer's disease patients). When it binds to beta sheet-rich structures, such as those in amyloid aggregates, the dye displays enhanced fluorescence. In the following experiments, the peptide KE₇, a naturally occurring core sequence of Amyloid-Beta(1-42), which forms amyloid aggregates, is used as the model system and positive control (see **Figures 2** and **18****).**

For the inhibition studies, KE₇ was mixed with inhibitor peptides in a 1:10 molar ratio (see **Figure 21 C)****.** The concentration of dye added was the same as that of the positive control, and the fluorescence signal was monitored over the course of an hour.

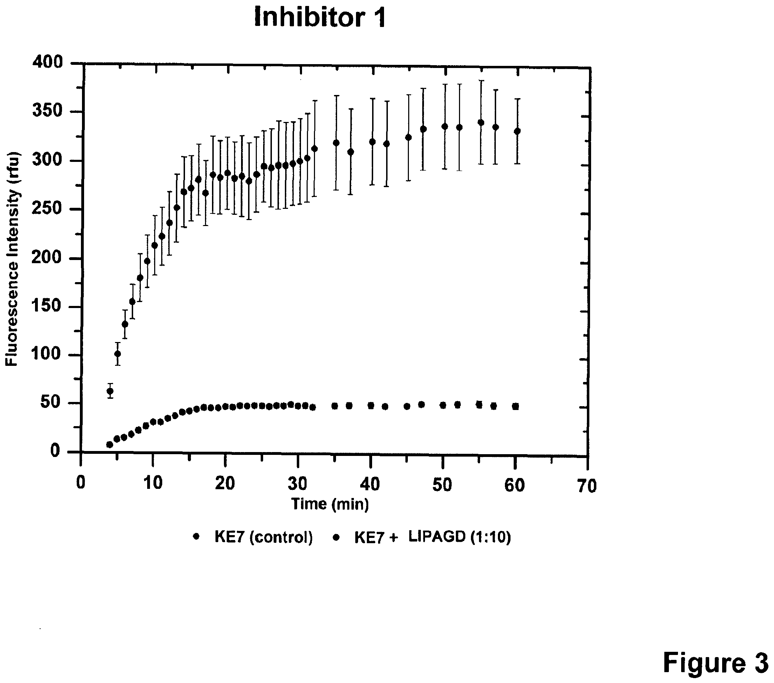

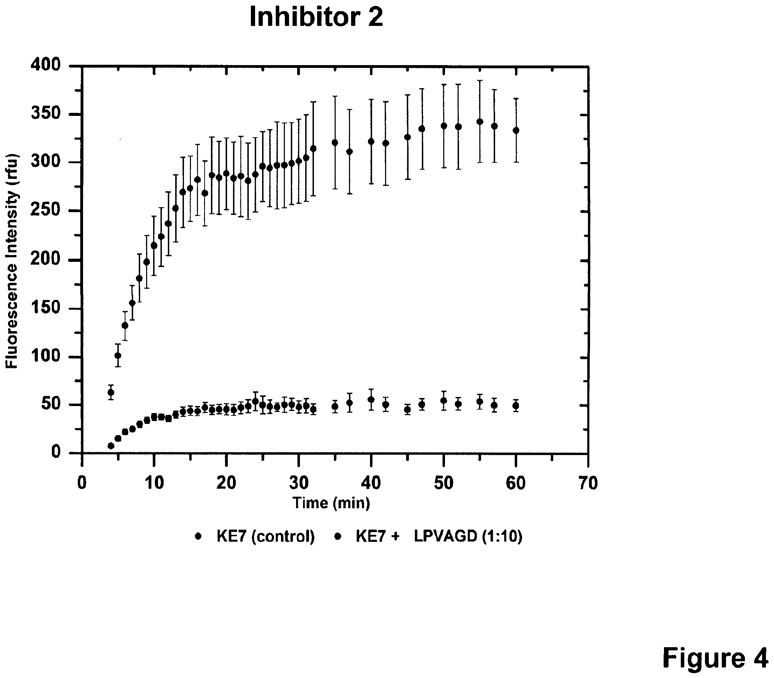

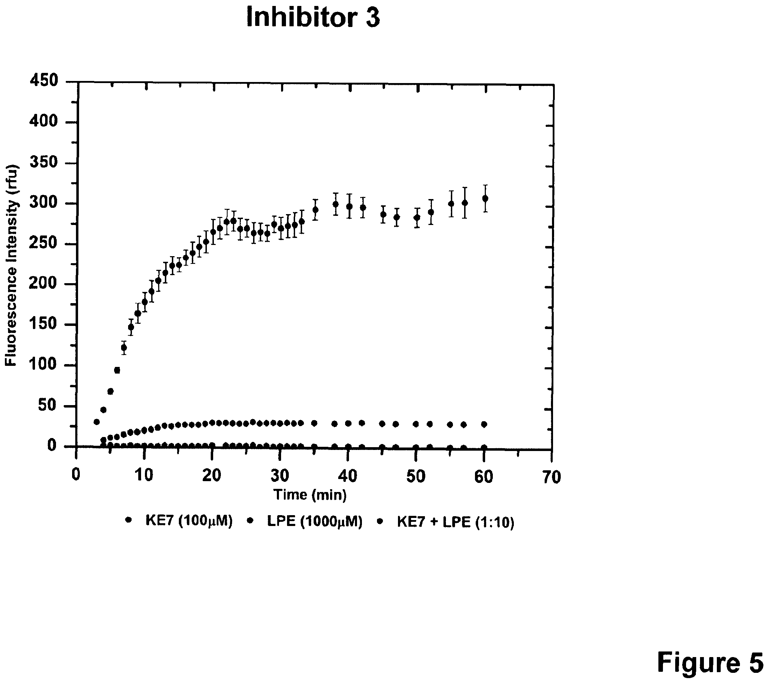

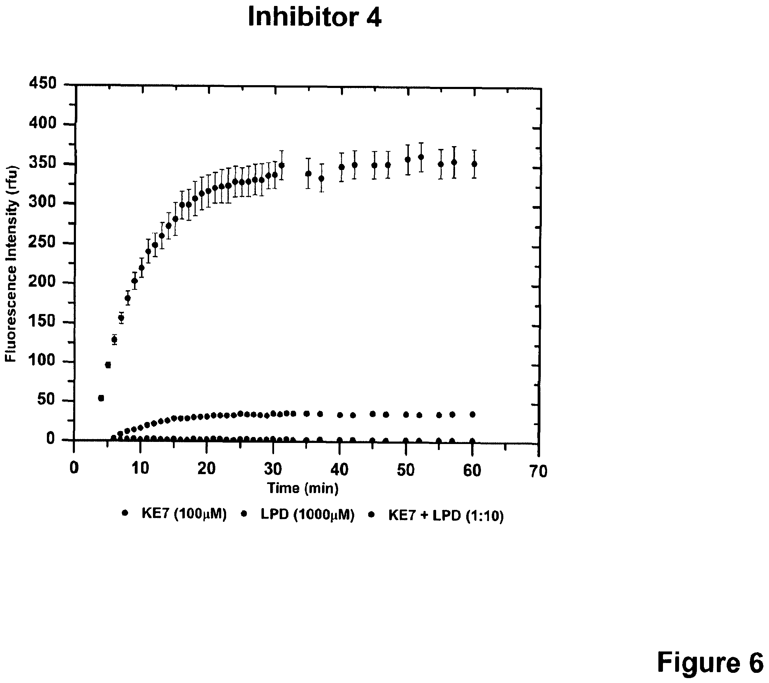

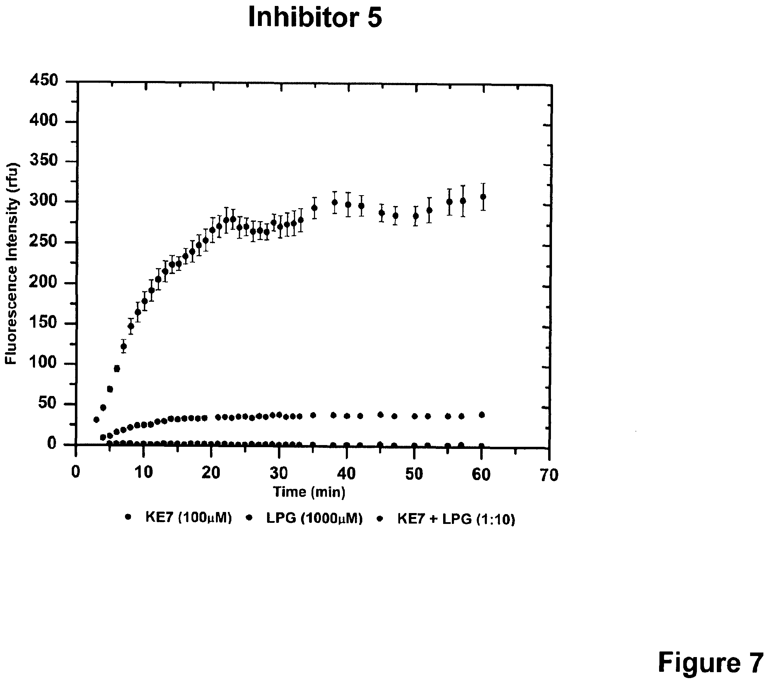

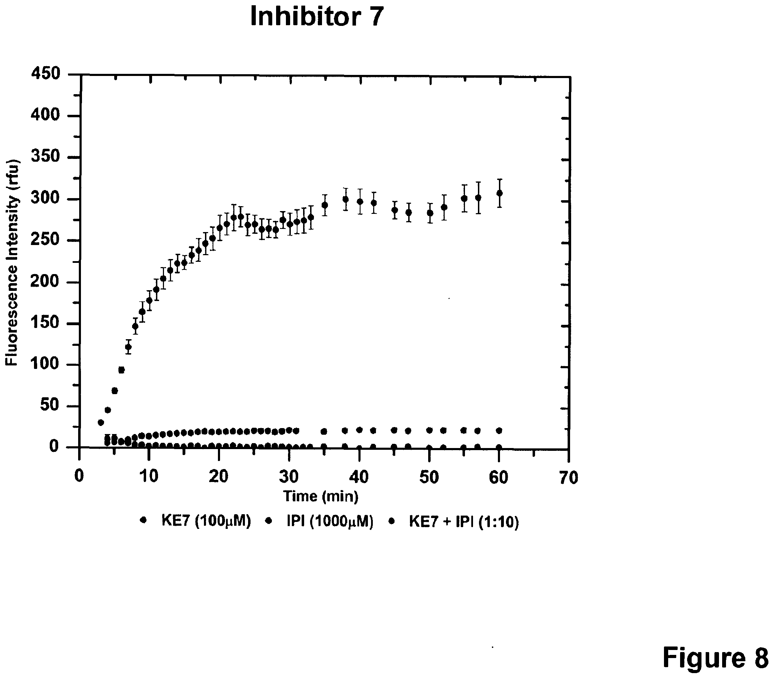

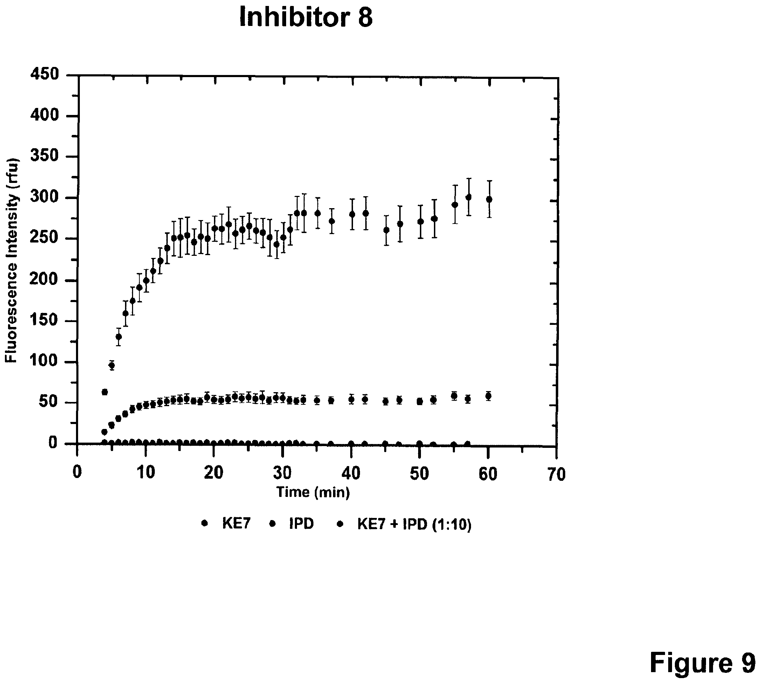

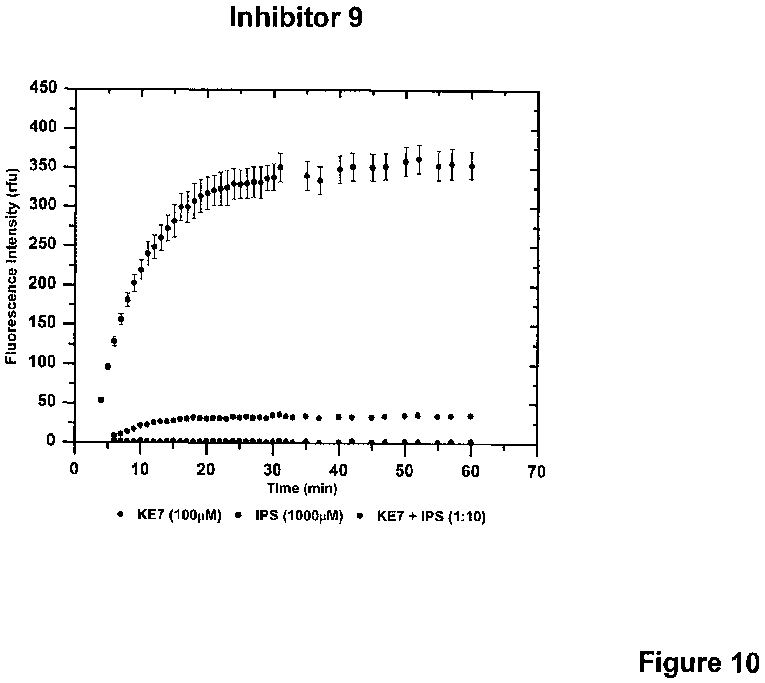

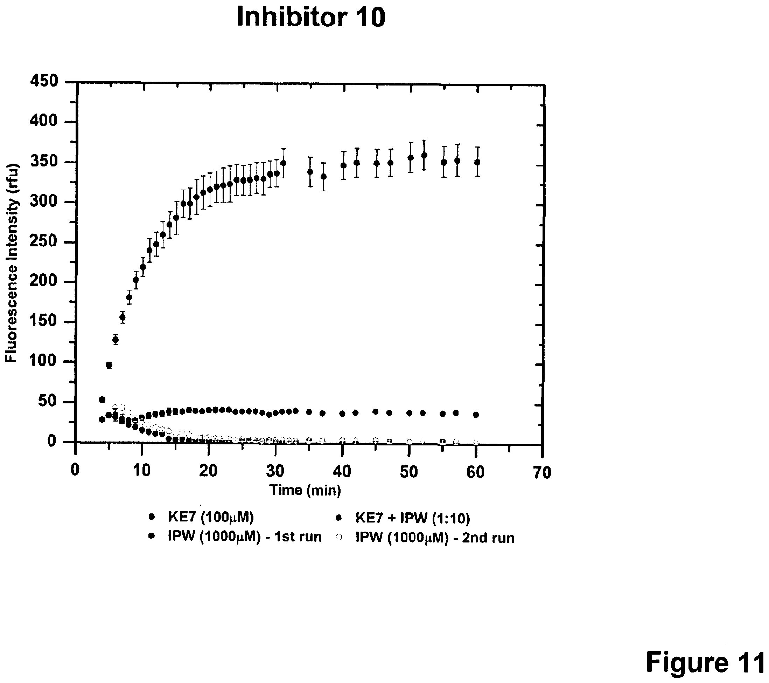

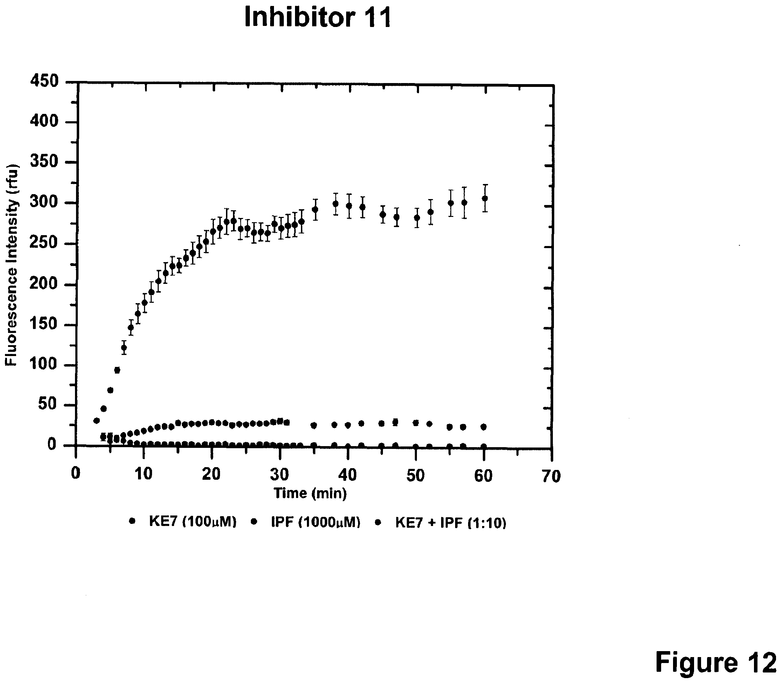

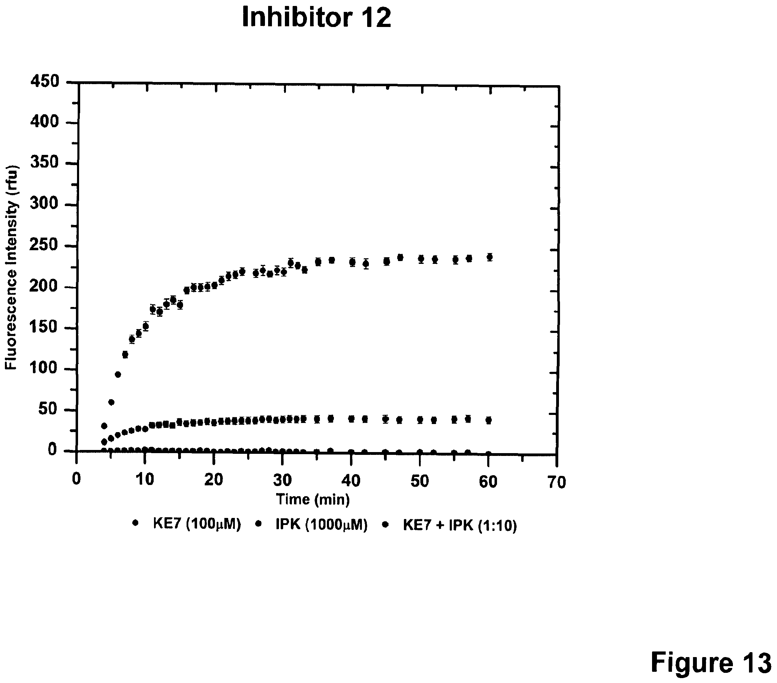

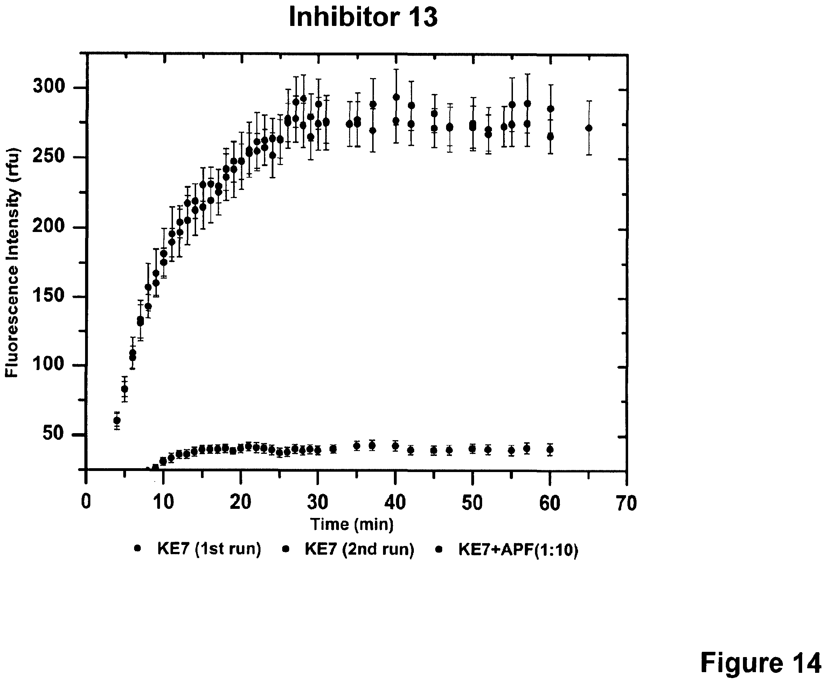

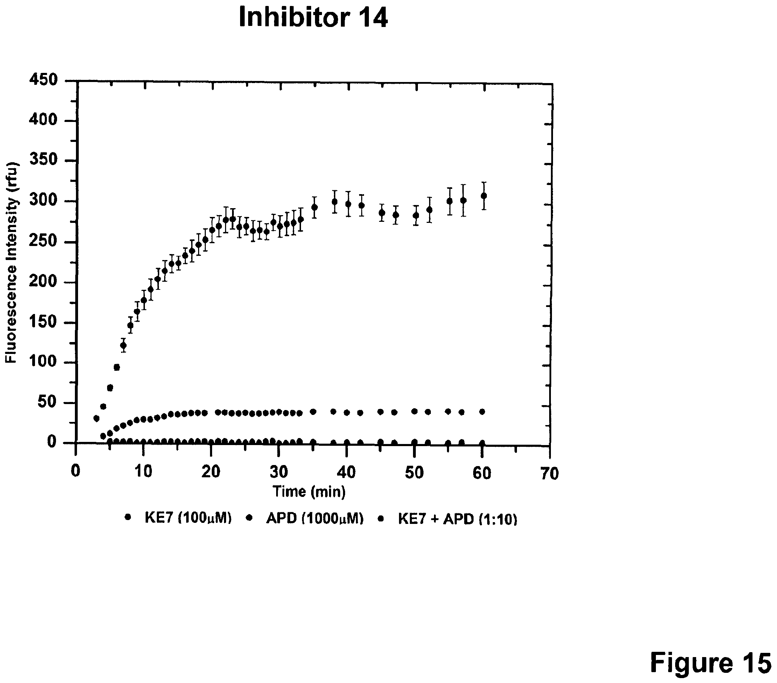

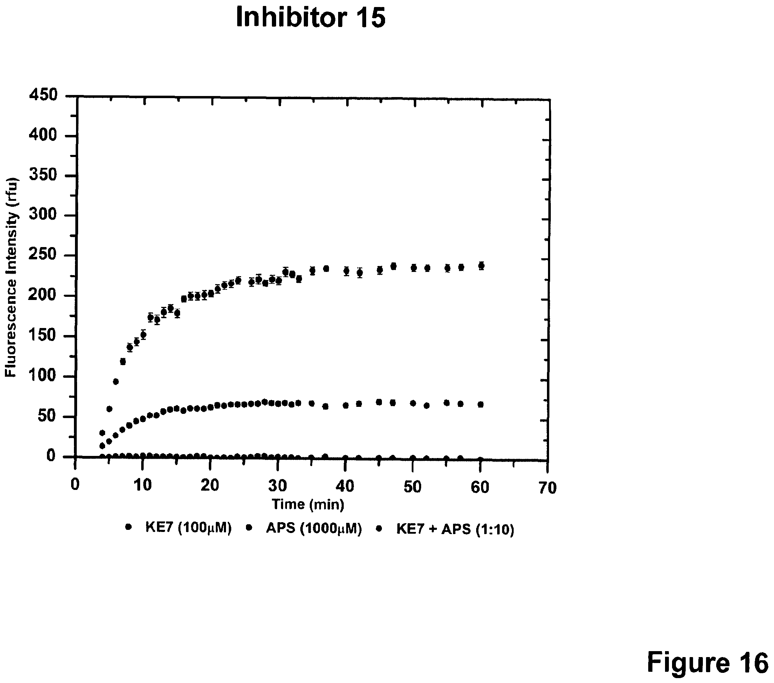

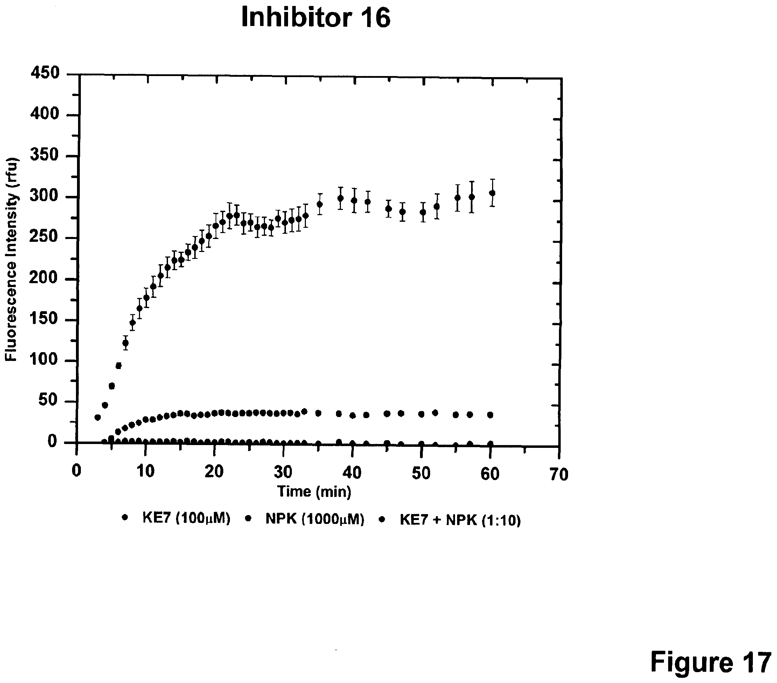

**Figures 3** to **17** as well as **Figures 21** **E** and **F** clearly show that the inhibitor peptides of the present invention, exemplified by inhibitor peptides 1 to 5 and 7 to 16, suppress KE₇ aggregation. While KE₇ without an inhibitor peptide shows a fluorescence enhancement due to the formation of amyloid fibrils, there is a marked decrease in the fluorescence signal once the inhibitor peptide is added. Inhibitors 1 to 16 correspond to individual inhibitor peptides from the list of SEQ ID NOs: 21 to 44 in the appended sequence listing.

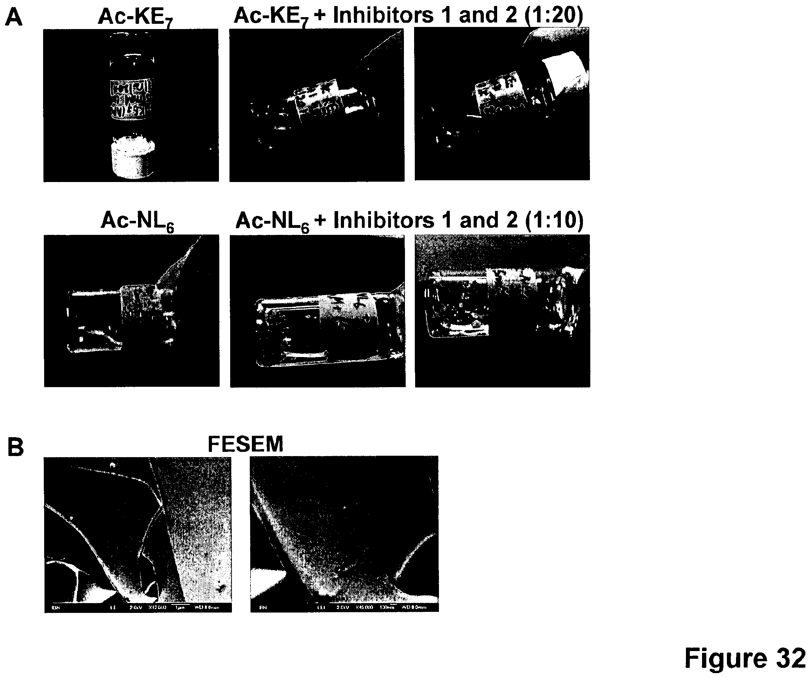

Furthermore, gelation studies with the peptides Ac-KE₇ and Ac-NL₆ in absence and presence of inhibitor peptides 1 and 2 showed that mixing with the inhibitor peptides prevented/inhibited hydrogel formation of the peptides Ac-KE₇ and Ac-NL₆ **(****Figure 32 A)****.**

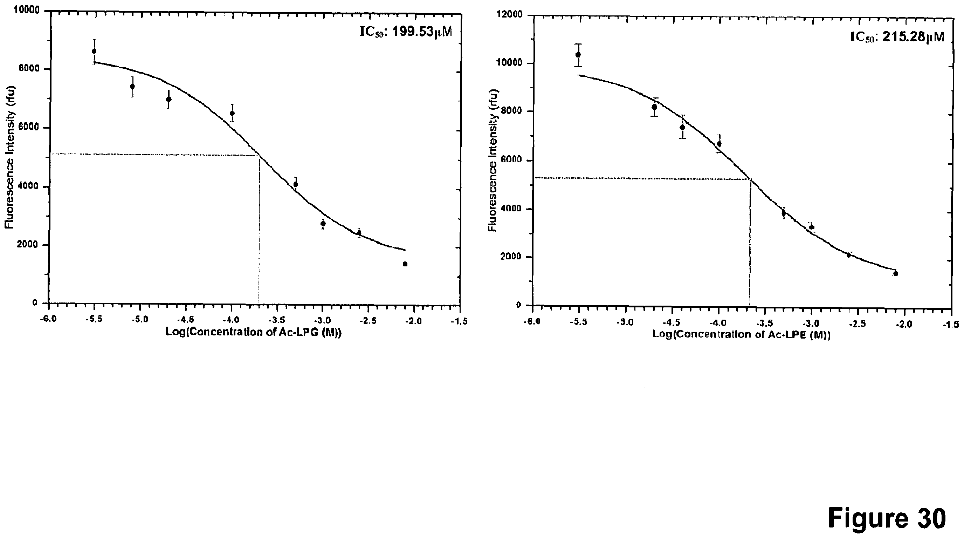

The half maximal inhibitory concentration (IC₅₀) is a measure of the effectiveness of a compound in inhibiting a particular biological process. This quantitative measure indicates how much of a particular drug or other substance (inhibitor) is needed to effectively prevent a given process, in this case amyloid aggregation. The IC₅₀ sigmoidal curves for the peptides Ac-LPE and Ac-LPG (inhibitor peptides 3 and 5, corresponding to SEQ ID NOs: 47 and 49; also referred to as Ac-LE₃ and Ac-LG₃) are shown in **Figure 30****.**

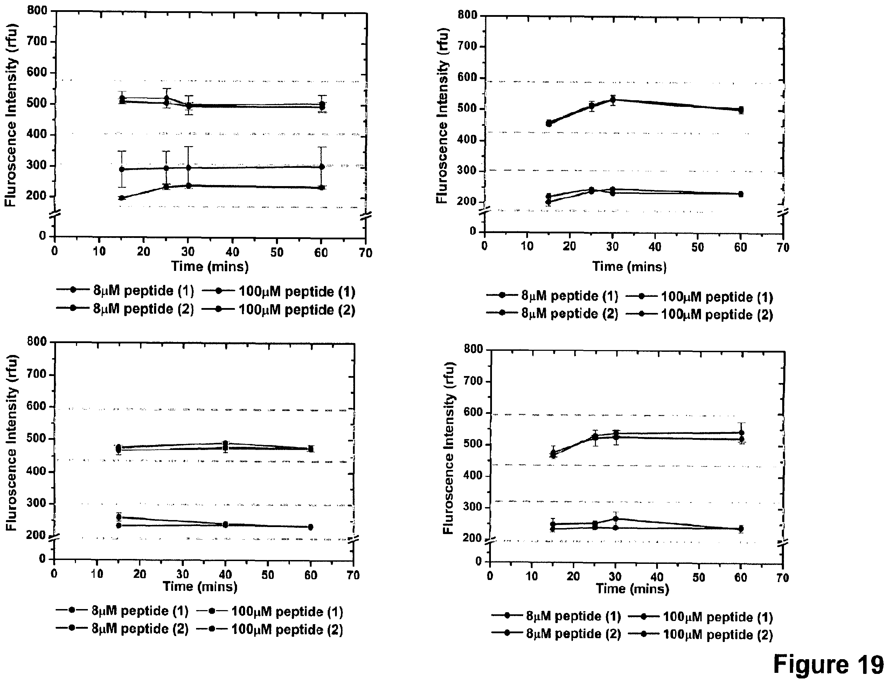

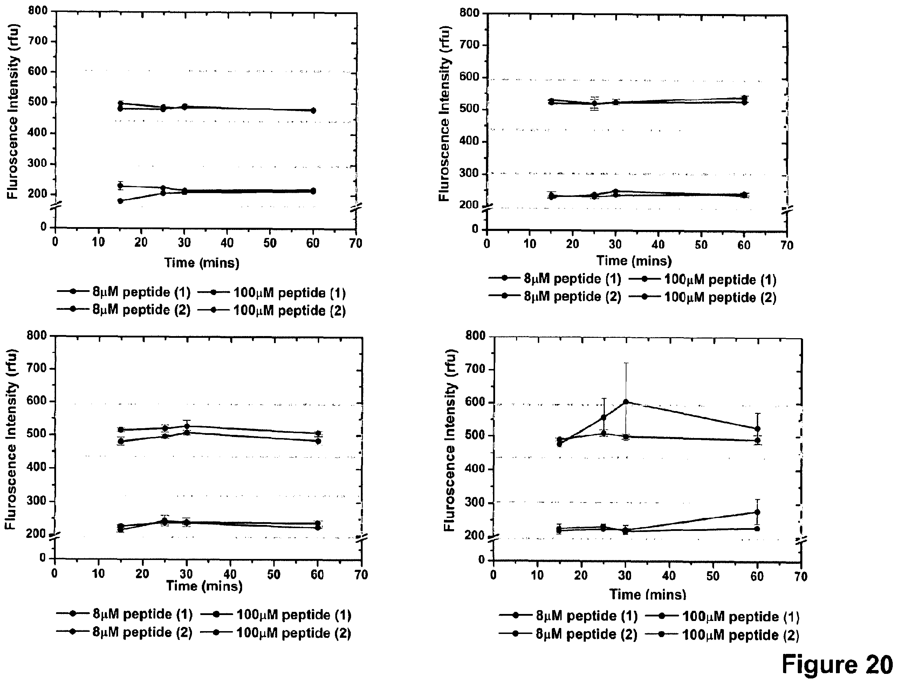

**Figures 19****,** **20** and **21** **D** show that, when the inhibitor peptide alone (concentrations of 8µm and 100 µm) is mixed with the dye, there is no fluorescence enhancement (values similar to those in the negative control range obtained with water + dye using a PMT Gain of 131).

The vials depicted in **Figure 21** **A** show that the pro line-containing inhibitor peptides according to the present invention can be dissolved in water. Good solubility in aqueous solutions is an advantage for drug development applications, especially when compared to aromatic compounds, which require organic solvents to dissolve completely.

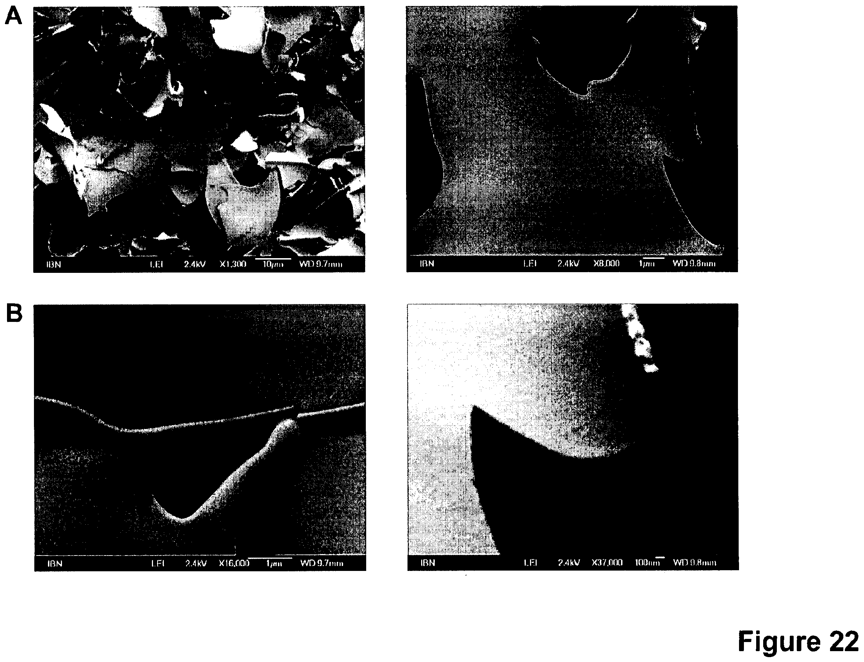

**Figures 22** **A** and **B** show the typical morphology of an inhibitor peptide according to the present invention at different magnifications using Field Emission Scanning Electron Microscopy (FESEM). A 10 mg/ml peptide solution was made by completely dissolving the inhibitor peptide in water. This solution was then shock frozen, lyophilized and coated with a thin layer of platinum to make the sample conductive for electron microscopy. Usually, amyloid fibers can be seen even under a normal optical microscope and in the case of amyloid core sequences that form hydrogels, fiber structures are clearly visible at magnifications starting from ∼ 3000x. For inhibitor peptide 1, no fibers were observed even at higher magnifications of 37,000x and more (see also **Figure 32 B)****.** In order for a peptide to be used as an effective inhibitor drug for amyloidosis, it is necessary to ensure that the inhibitor can effectively recognize and bind to natural amyloids/growing amyloid fibrils without itself forming fibers.

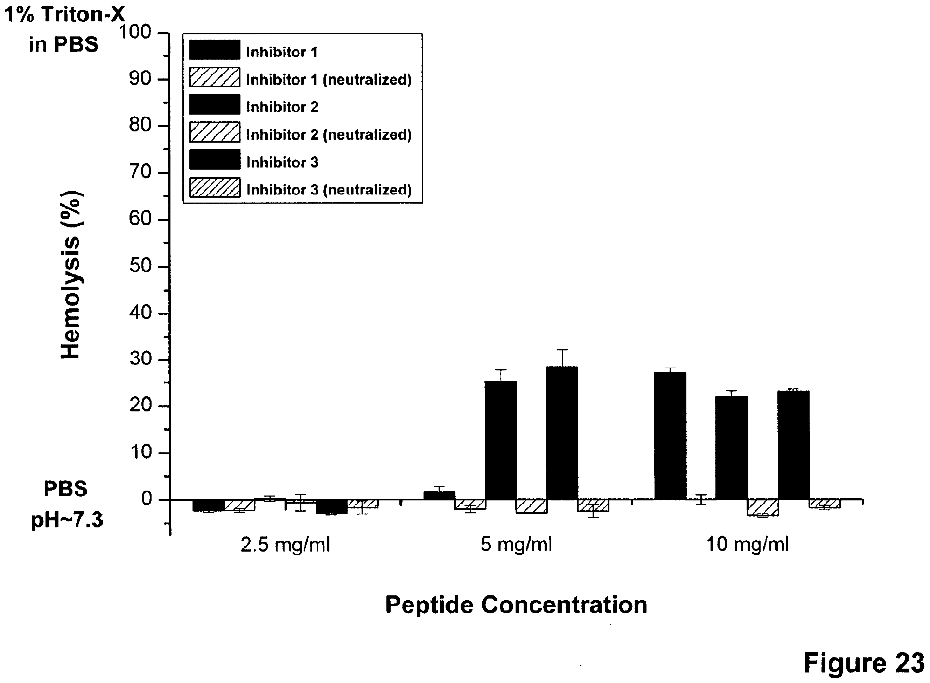

**Figure 23** shows the results of a hemolysis assay testing the biocompatibility of the inhibitors. Any drug candidate has to be first tested for biocompatibility with blood and biological tissue. Here, a hemolysis assay was performed to determine whether the inhibitor peptides had any adverse effect on red blood cells. Three of the inhibitor peptides were used at concentrations of 2.5, 5 and 10 mg/ml in water. The pH of these solutions were measured and it was found that samples with a higher concentration of the peptides were acidic (pH <4). Thus, a set of neutralized peptide solutions (neutralized with 1% w/v NaOH) was prepared at the same concentrations for comparison. No lysis was observed with low peptide concentrations (2.5 mg/ml) and with neutralized solutions of the inhibitor peptides, indicating that the 25% lysis observed for higher concentrations of 5 and 10 mg/ml was due to the acidic pH rather than the peptide itself.

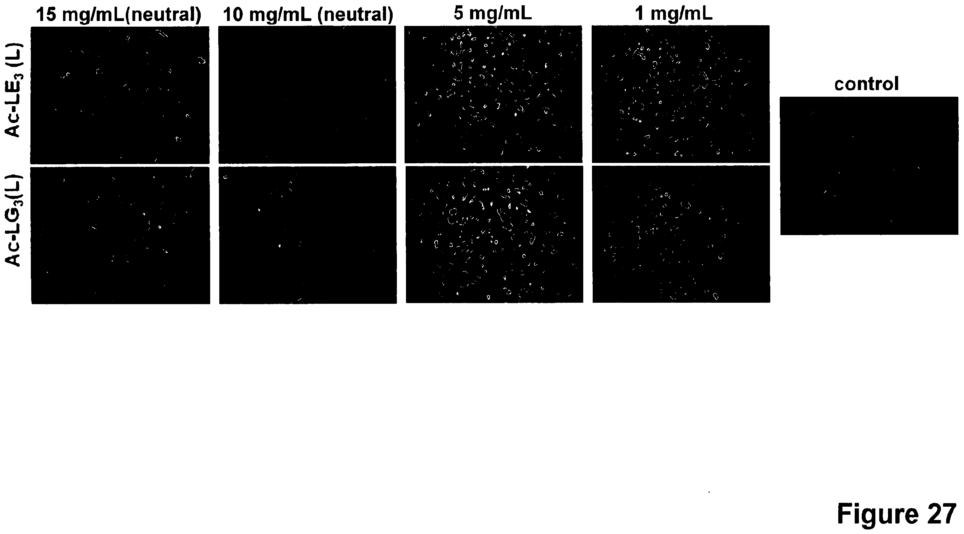

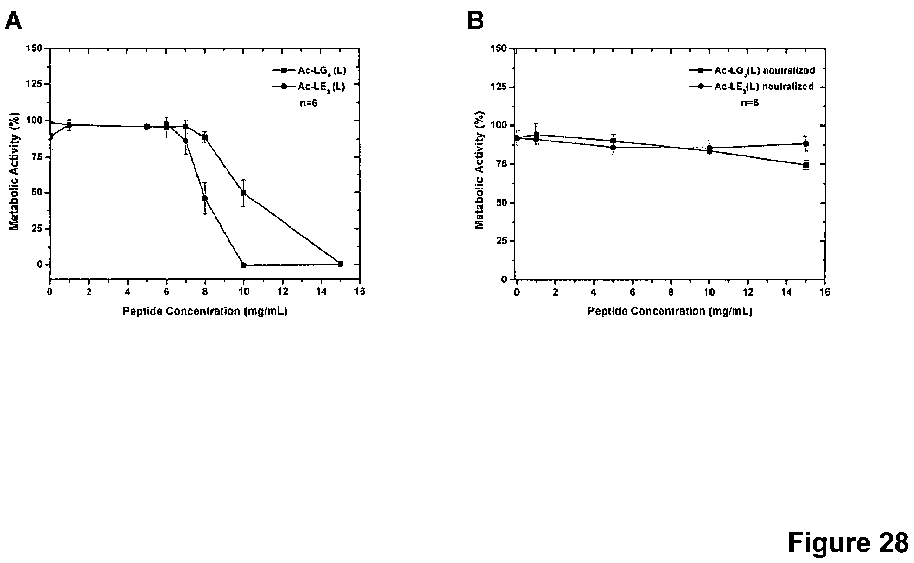

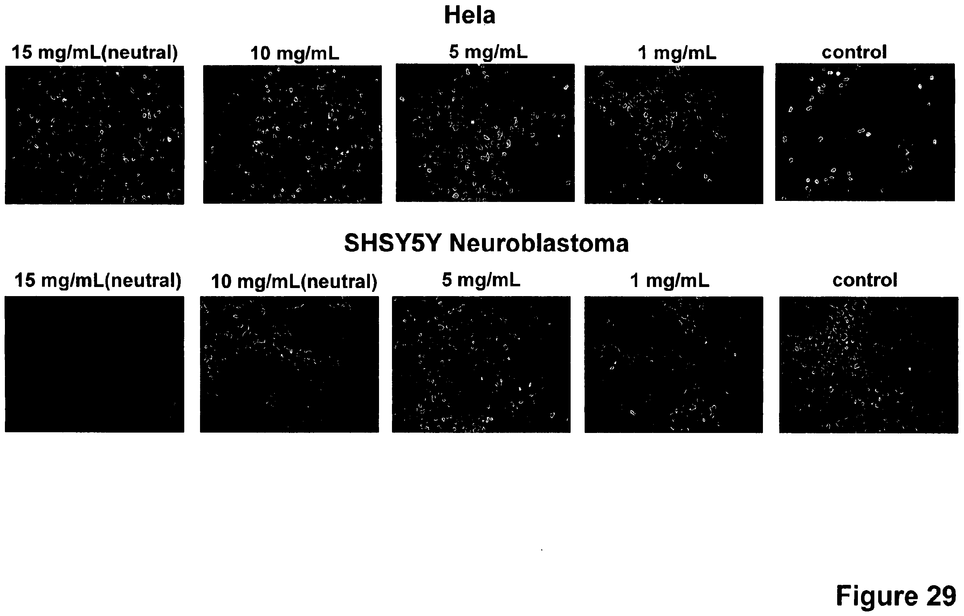

Similar results were obtained in a WST-1 assay and live/dead cytotoxicity assays using various cell lines (see Figures **27** to **29****).**

Influence of salt concentration on the mechanical stability of peptide-derived hydrogels

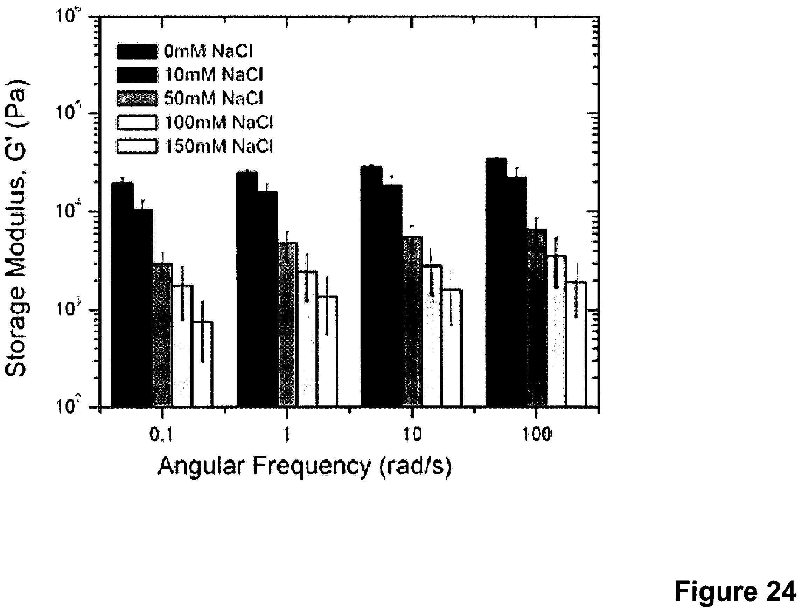

**Figure 24** shows that increasing the concentration of NaCl decreased the storage modulus G' / mechanical strength of hydrogels derived from 10 mg/mL of Ac-LD₆ (L).

The features of the present invention disclosed in the specification, the claims, and/or in the accompanying drawings may, both separately and in any combination thereof, be material for realizing the invention in various forms thereof.

## Claims

1. An isolated alpha-helix breaking peptide for use in the treatment of a disease associated with amyloidosis, said isolated alpha-helix breaking peptide having the general formula
Z-(X)ₘ-Proline-(X)ₙ,
wherein
Z is an N-terminal protecting group;
X is, at each occurrence, independently selected from non-aromatic amino acids; and m and n indicate the number of amino acids and are integers independently selected from 1 to 5, with m + n being ≤ 6,
wherein said N-terminal protecting group has the general formula -C(O)-R, R being selected from the group consisting of methyl, ethyl, propyl, isopropyl, butyl and isobutyl,
and wherein the N-terminal amino acid of said peptide is more hydrophobic than the C-terminal amino acid of said peptide,
wherein said disease associated with amyloidosis is selected from the group consisting of Neuro-degenerative diseases, such as Alzheimer's disease, Parkinson's disease, Huntington's disease, Amyotrophic lateral sclerosis (ALS) and Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia 17, Spinal and bulbar muscular atrophy, Hereditary dentatorubral-pallidoluysian atrophy, Familial British dementia, Familial Danish dementia, Non-neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAI amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis.

2. The isolated alpha-helix breaking peptide for use in the treatment of a disease associated with amyloidosis according to claim 1, wherein X is, at each occurrence, independently selected from naturally occurring amino acids.

3. The isolated alpha-helix breaking peptide for use in the treatment of a disease associated with amyloidosis according to claim 1 or 2, wherein at least one X is a D-amino acid.

4. The isolated alpha-helix breaking peptide for use in the treatment of a disease associated with amyloidosis according to any of the foregoing claims, wherein said non-aromatic amino acids are selected from the group consisting of isoleucine, leucine, valine, alanine, glycine, aspartic acid, asparagine, glutamic acid, glutamine, serine, threonine and lysine.

5. The isolated alpha-helix breaking peptide for use in the treatment of a disease associated with amyloidosis according to any of the foregoing claims, wherein m + n is ≤ 5, preferably ≤ 4, more preferably ≤ 3.

6. The isolated alpha-helix breaking peptide for use in the treatment of a disease associated with amyloidosis according to any of the foregoing claims, wherein m and n are 1.

7. The isolated alpha-helix breaking peptide for use in the treatment of a disease associated with amyloidosis according to any of the foregoing claims, wherein said N-terminal protecting group is an acetyl group.

8. The isolated alpha-helix breaking peptide for use in the treatment of a disease associated with amyloidosis according to any of the foregoing claims, wherein the C-terminus of said peptide is amidated or esterified, wherein, preferably, the C-terminus has the formula -CONHR, with R being selected from the group consisting of H, alkyl and substituted alkyl, or the formula -COOR, with R being selected from the group consisting of alkyl and substituted alkyl.

9. The isolated alpha-helix breaking peptide for use in the treatment of a disease associated with amyloidosis according to any of the foregoing claims, wherein said peptide is provided in an aqueous solution, optionally comprising a physiological buffer.

10. The isolated alpha-helix breaking peptide for use in the treatment of a disease associated with amyloidosis according to any of the foregoing claims, wherein said peptide is administered orally.

11. A pharmaceutical composition for use in the treatment of a disease associated with amyloidosis comprising an isolated alpha-helix breaking peptide according to any of the foregoing claims,
wherein said disease associated with amyloidosis is selected from the group consisting of Neuro-degenerative diseases, such as Alzheimer's disease, Parkinson's disease, Huntington's disease, Amyotrophic lateral sclerosis (ALS) and Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia 17, Spinal and bulbar muscular atrophy, Hereditary dentatorubral-pallidoluysian atrophy, Familial British dementia, Familial Danish dementia, Non-neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAI amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis.

12. The pharmaceutical composition for use in the treatment of a disease associated with amyloidosis according to claim 11, further comprising at least one pharmaceutically acceptable carrier, diluent and/or excipient.

13. The pharmaceutical composition for use in the treatment of a disease associated with amyloidosis according to claim 11 or 12, further comprising a monovalent or divalent metal salt.

14. The pharmaceutical composition for use in the treatment of a disease associated with amyloidosis according to claim 13, wherein said metal is selected from the group consisting of sodium, magnesium, calcium and zinc.

## Patentansprüche

1. Isoliertes, alpha-Helix-brechendes Peptid zur Verwendung bei der Behandlung einer Krankheit, die mit Amyloidose assoziiert ist, wobei das isolierte alpha-Helix-brechende Peptid die allgemeine Formel hat
Z-(X)ₘ-Prolin-(X)ₙ,
wobei
Z eine N-terminale Schutzgruppe ist;
X bei jedem Auftreten unabhängig ausgewählt ist aus nicht-aromatischen Aminosäuren; und m und n die Anzahl der Aminosäuren anzeigen und ganze Zahlen sind, unabhängig ausgewählt aus 1 bis 5, wobei m + n ≤ 6,
wobei die N-terminale Schutzgruppe die allgemeine Formel -C(O)-R hat, wobei R ausgewählt ist aus der Gruppe, bestehend aus Methyl, Ethyl, Propyl, Isopropyl, Butyl und Isobutyl,
und wobei die N-terminale Aminosäure des Peptids hydrophober als die C-terminale Aminosäure des Peptids ist,
wobei die mit Amyloidose assoziierte Krankheit ausgewählt ist aus der Gruppe, bestehend aus neurodegenerativen Erkrankungen, wie etwa Alzheimer'sche Krankheit, Parkinson'sche Krankheit, Huntington'sche Krankheit, Amyotrophe Lateralsklerose (ALS) und Prion-bezogene oder spongiforme Enzephalopathien, wie etwa Creutzfeld-Jakob, Lewy-Körperchen-Demenz, Frontotemporale Demenz mit Parkinsonismus, Spinozerebelläre Ataxien, Spinozerebelläre Ataxie 17, Spinobulbäre Muskelatrophie, erbliche Dentatorubro-Pallidoluysische Atrophie, Familiäre Britische Demenz, Familiäre Dänische Demenz, nichtneuropathisch lokalisierte Erkrankungen wie etwa Diabetes Mellitus vom Typ II, Medulläres Karzinom der Schilddrüse, Atrium-Amyloidose, erbliche zerebrale Blutung mit Amyloidose, Hypophysen-Prolactinom, Injektionslokalisierte Amyloidose, Aorten-medial-Amyloidose, erbliche Gitter-Netzhaut-Dystrophie, Netzhautamyloidose, die mit Trichiasis assoziiert ist, Katarakt, Kalzifizierende Epithel-odontogene Tumore, Pulmonale Alveolarproteinose, Inclusion-Body-Myositis, Kutane Liehen Amyloidosus, und nichtneuropathische systemische Amyloidose, wie etwas AL-Amyloidose, AA-Amyloidose, Familiäres Mittelmeerfieber, senile systemische Amyloidose, Familäre Amyloidotische Polyneuropathie, Hämodialyse-verwandte Amyloidose, ApoAI Amyloidose, ApoAII Amyloidose, ApoAIV Amyloidose, erbliche Finnische Amyloidose, Lysozym-Amyloidose, Fibrinogen-Amyloidose, erbliche Isländische zerebrale amyloide Angiopathie, Familiäre Amyloidose und systemische Amyloidose, die in vielfachen Geweben auftritt, wie etwa Leichtlcetten-Amyloidose.

2. Isoliertes, alpha-Helix-brechendes Peptid zur Verwendung bei der Behandlung einer Krankheit, die mit Amyloidose assoziiert ist, gemäß Anspruch 1, wobei X bei jedem Auftreten unabhängig ausgewählt ist aus natürlich vorkommenden Aminosäuren.

3. Isoliertes, alpha-Helix-brechendes Peptid zur Verwendung bei der Behandlung einer Krankheit, die mit Amyloidose assoziiert ist, nach Anspruch 1 oder 2, wobei wenigstens ein X eine D-Aminosäure ist.

4. Isoliertes, alpha-Helix-brechendes Peptid zur Verwendung bei der Behandlung einer Krankheit, die mit Amyloidose assoziiert ist, nach einem der vorangehenden Ansprüche, wobei die nicht-aromatischen Aminosäuren ausgewählt sind aus der Gruppe, bestehend aus Isoleucin, Leucin, Valin, Alanine, Glycin, Asparaginsäure, Asparagin, Glutaminsäure, Glutamin, Serin, Threonin und Lysin.

5. Isoliertes, alpha-Helix-brechendes Peptid zur Verwendung bei der Behandlung einer Krankheit, die mit Amyloidose assoziiert ist, nach einem der vorangehenden Ansprüche, wobei m + n ≤ 5, bevorzugt ≤4, bevorzugter ≤3 ist.

6. Isoliertes, alpha-Helix-brechendes Peptid zur Verwendung bei der Behandlung einer Krankheit, die mit Amyloidose assoziiert ist, nach einem der vorangehenden Ansprüche, wobei m und n 1 sind.

7. Isoliertes, alpha-Helix-brechendes Peptid zur Verwendung bei der Behandlung einer Krankheit, die mit Amyloidose assoziiert ist, nach einem der vorangehenden Ansprüche, wobei die N-terminale Schutzgruppe eine Acetyl Gruppe ist.

8. Isoliertes, alpha-Helix-brechendes Peptid zur Verwendung bei der Behandlung einer Krankheit, die mit Amyloidose assoziiert ist, nach einem der vorangehenden Ansprüche, wobei der C-Terminus des Peptids amidiert oder verestert ist, wobei bevorzugt der C-Terminus die Formel -CONHR, wobei R ausgewählt ist aus der Gruppe, bestehend aus H, Alkyl und substituiertem Alkyl, oder die Formel -COOR hat, wobei R ausgewählt ist aus der Gruppe, bestehend aus Alkyl und substituiertem Alkyl.

9. Isoliertes, alpha-Helix-brechendes Peptid zur Verwendung bei der Behandlung einer Krankheit, die mit Amyloidose assoziiert ist, nach einem der vorangehenden Ansprüche, wobei das Peptid in einer wässrigen Lösung bereitgestellt wird, die fakultativ einen physiologischen Puffer umfasst.

10. Isoliertes, alpha-Helix-brechendes Peptid zur Verwendung bei der Behandlung einer Krankheit, die mit Amyloidose assoziiert ist, nach einem der vorangehenden Ansprüche, wobei das Peptid oral verabreicht wird.

11. Pharmazeutische Zusammensetzung zur Verwendung bei der Behandlung einer Krankheit, die mit Amyloidose assoziiert ist, umfassend ein isoliertes alpha-Helix-brechendes Peptid nach einem der vorangehenden Ansprüche, wobei die mit Amyloidose assoziierte Erkrankung ausgewählt ist aus der Gruppe, bestehend aus neurodegenerativen Erkrankungen, wie etwa Alzheimer'sche Krankheit, Parkinson'sche Krankheit, Huntington'sche Krankheit, Amyotrophe Lateralsklerose (ALS) und Prion-bezogene oder spongiforme Enzephalopathien, wie etwa Creutzfeld-Jakob, Lewy-Körperchen-Demenz, Frontotemporale Demenz mit Parkinsonismus, Spinozerebelläre Ataxien, Spinozerebelläre Ataxie 17, Spinobulbäre Muskelatrophie, erbliche Dentatorubro-Pallidoluysische Atrophie, Familiäre Britische Demenz, Familiäre Dänische Demenz, nichtneuropathisch lokalisierte Erkrankungen wie etwa Diabetes Mellitus vom Typ II, Medulläres Karzinom der Schilddrüse, Atrium-Amyloidose, erbliche zerebrale Blutung mit Amyloidose, Hypophysen-Prolactinom, Injektionslokalisierte Amyloidose, Aorten-medial-Amyloidose, erbliche Gitter-Netzhaut-Dystrophie, Netzhautamyloidose, die mit Trichiasis assoziiert ist, Katarakt, Kalzifizierende Epithel-odontogene Tumore, Pulmonale Alveolarproteinose, Inclusion-Body-Myositis, Kutane Lichen Amyloidosus, und nichtneuropathische systemische Amyloidose, wie etwas AL-Amyloidose, AA-Amyloidose, Familiäres Mittelmeerfieber, senile systemische Amyloidose, Familäre Amyloidotische Polyneuropathie, Hämodialyse-verwandte Amyloidose, ApoAI Amyloidose, ApoAII Amyloidose, ApoAIV Amyloidose, erbliche Finnische Amyloidose, Lysozym-Amyloidose, Fibrinogen-Amyloidose, erbliche Isländische zerebrale amyloide Angiopathie, Familiäre Amyloidose und systemische Amyloidose, die in vielfachen Geweben auftritt, wie etwa Leichtlcetten-Amyloidose.

12. Pharmazeutische Zusammensetzung zur Verwendung bei der Behandlung einer Krankheit, die mit Amyloidose assoziiert ist, nach Anspruch 11, weiterhin umfassend wenigstens einen pharmazeutisch akzeptablen Träger, Verhütungsmittel und/oder Hilfsstoff.

13. Pharmazeutische Zusammensetzung zur Verwendung bei der Behandlung einer Krankheit, die mit Amyloidose assoziiert ist, nach Anspruch 11 oder 12, weiterhin umfassend eine monovalentes oder bivalentes Metallsalz.

14. Pharmazeutische Zusammensetzung zur Verwendung bei der Behandlung einer Krankheit, die mit Amyloidose assoziiert ist, nach Anspruch 13, wobei das Metall ausgewählt ist aus der Gruppe, bestehend aus Natrium, Magnesium, Calcium und Zink.

## Revendications

1. Peptide de rupture d'hélice alpha isolé pour son utilisation dans le traitement d'une maladie associée à une amyloïdose, ledit peptide de rupture d'hélice alpha isolé possédant la formule générale
*Z*-(X)ₘ-Proline-(X)ₙ,
dans laquelle
Z est un groupe protecteur N-terminal ;
X est, à chaque occurrence, choisi indépendamment parmi les acides aminés non aromatiques ; et
m et n indiquent le nombre d'acides aminés et sont des entiers choisis indépendamment parmi 1 à 5, avec m + n ≤ 6,
dans lequel ledit groupe protecteur N-terminal possède la formule générale -C(O)-R, R étant choisi dans le groupe constitué par les groupes méthyle, éthyle, propyle, isopropyle, butyle et isobutyle,
et dans lequel l'acide aminé N-terminal dudit peptide est plus hydrophobe que l'acide aminé C-terminal dudit peptide,
dans lequel ladite maladie associée à une amyloïdose est choisie dans le groupe constitué par les maladies neurodégénératives, telles que la maladie d'Alzheimer, la maladie de Parkinson, la maladie de Huntington, la sclérose latérale amyotrophique (SLA) et les encéphalopathies associées aux prions ou spongiformes, telles que la maladie de Creutzfeld-Jacob, la démence à corps de Lewy, la démence fronto-temporale avec syndrome parkinsonien, les ataxies spinocérébelleuses, l'ataxie spinocérébelleuses 17, l'atrophie musculaire spinale et bulbaire, l'atrophie dentaturo-rubro-pallido-luysienne héréditaire, la démence britannique familiale, la démence danoise familiale, les maladies localisées non neuropathiques, telles que le diabète sucré de type II, le carcinome médullaire de la thyroïde, l'amyloïdose atriale, les hémorragies cérébrales héréditaires avec amyloïdose, le prolactinome hypophysaire, l'amyloïdose localisée par injection, l'amyloïdose médiale aortique, la dystrophie cornéenne grillagée héréditaire, l'amyloïdose cornéenne associée au trichiasis, la cataracte, les tumeurs odontogènes épithéliales calcifiantes, la protéinose pulmonaire alvéolaire, la myosite à corps d'inclusion, le lichen amyloïde cutané, et une amyloïdose systémique non neuropathique, telle que l'amyloïdose AL, l'amyloïdose AA, la fièvre méditerranéenne familiale, l'amyloïdose systémique sénile, la polyneuropathie familiale amyloïde, l'amyloïdose associée à l'hémodialyse, l'amyloïdose ApoAI, l'amyloïdose ApoAII, l'amyloïdose ApoAIV, l'amyloïdose finnoise héréditaire, l'amyloïdose à lysozyme, l'amyloïdose à fibrinogène, l'angiopathie amyloïde cérébrale islandaise héréditaire, l'amyloïdose familiale, et l'amyloïdose systémique qui apparaît dans de multiples tissus, telle que l'amyloïdose à chaîne légère.

2. Peptide de rupture d'hélice alpha isolé pour son utilisation dans le traitement d'une maladie associée à une amyloïdose selon la revendication 1, dans lequel X est, à chaque occurrence, choisi indépendamment parmi les acides aminés d'origine naturelle.

3. Peptide de rupture d'hélice alpha isolé pour son utilisation dans le traitement d'une maladie associée à une amyloïdose selon la revendication 1 ou 2, dans lequel au moins un X est un acide aminé D.

4. Peptide de rupture d'hélice alpha isolé pour son utilisation dans le traitement d'une maladie associée à une amyloïdose selon l'une quelconque des revendications précédentes, dans lequel lesdits acides aminés non aromatiques sont choisis dans le groupe constitué par l'isoleucine, la leucine, la valine, l'alanine, la glycine, l'acide aspartique, l'asparagine, l'acide glutamique, la glutamine, la sérine, la thréonine et la lysine.

5. Peptide de rupture d'hélice alpha isolé pour son utilisation dans le traitement d'une maladie associée à une amyloïdose selon l'une quelconque des revendications précédentes, dans lequel m +n ≤ 5, de préférence ≤ 4, de manière davantage préférée ≤ 3.

6. Peptide de rupture d'hélice alpha isolé pour son utilisation dans le traitement d'une maladie associée à une amyloïdose selon l'une quelconque des revendications précédentes, dans lequel m et n sont 1.

7. Peptide de rupture d'hélice alpha isolé pour son utilisation dans le traitement d'une maladie associée à une amyloïdose selon l'une quelconque des revendications précédentes, dans lequel ledit groupe protecteur N-terminal est un groupe acétyle.

8. Peptide de rupture d'hélice alpha isolé pour son utilisation dans le traitement d'une maladie associée à une amyloïdose selon l'une quelconque des revendications précédentes, dans lequel l'extrémité C-terminale dudit peptide est amidée ou estérifiée, dans lequel, de préférence, l'extrémité C-terminale a la formule -CONHR, R étant choisi dans le groupe constitué par H, un alkyle et un alkyle substitué, ou la formule -COOR, R étant choisi dans le groupe constitué par un alkyle et un alkyle substitué.

9. Peptide de rupture d'hélice alpha isolé pour son utilisation dans le traitement d'une maladie associée à une amyloïdose selon l'une quelconque des revendications précédentes, dans lequel ledit peptide est fourni dans une solution aqueuse, comprenant facultativement un tampon physiologique.

10. Peptide de rupture d'hélice alpha isolé pour son utilisation dans le traitement d'une maladie associée à une amyloïdose selon l'une quelconque des revendications précédentes, dans lequel ledit peptide est administré par voie orale.

11. Composition pharmaceutique pour son utilisation dans le traitement d'une maladie associée à une amyloïdose comprenant un peptide de rupture d'hélice alpha isolé selon l'une quelconque des revendications précédentes,
dans laquelle ladite maladie associée à une amyloïdose est choisie dans le groupe constitué par les maladies neurodégénératives, telles que la maladie d'Alzheimer, la maladie de Parkinson, la maladie de Huntington, la sclérose latérale amyotrophique (SLA) et les encéphalopathies associées aux prions ou spongiformes, telles que la maladie de Creutzfeld-Jacob, la démence à corps de Lewy, la démence fronto-temporale avec syndrome parkinsonien, les ataxies spinocérébelleuses, l'ataxie spinocérébelleuses 17, l'atrophie musculaire spinale et bulbaire, l'atrophie dentaturo-rubro-pallido-luysienne héréditaire, la démence britannique familiale, la démence danoise familiale, les maladies localisées non neuropathiques, telles que le diabète sucré de type II, le carcinome médullaire de la thyroïde, l'amyloïdose atriale, les hémorragies cérébrales héréditaires avec amyloïdose, le prolactinome hypophysaire, l'amyloïdose localisée par injection, l'amyloïdose médiale aortique, la dystrophie cornéenne grillagée héréditaire, l'amyloïdose cornéenne associée au trichiasis, la cataracte, les tumeurs odontogènes épithéliales calcifiantes, la protéinose pulmonaire alvéolaire, la myosite à corps d'inclusion, le lichen amyloïde cutané, et une amyloïdose systémique non neuropathique, telle que l'amyloïdose AL, l'amyloïdose AA, la fièvre méditerranéenne familiale, l'amyloïdose systémique sénile, la polyneuropathie familiale amyloïde, l'amyloïdose associée à l'hémodialyse, l'amyloïdose ApoAI, l'amyloïdose ApoAII, l'amyloïdose ApoAIV, l'amyloïdose finnoise héréditaire, l'amyloïdose à lysozyme, l'amyloïdose à fibrinogène, l'angiopathie amyloïde cérébrale islandaise héréditaire, l'amyloïdose familiale, et l'amyloïdose systémique qui apparaît dans de multiples tissus, telle que l'amyloïdose à chaîne légère.

12. Composition pharmaceutique pour son utilisation dans le traitement d'une maladie associée à une amyloïdose selon la revendication 11, comprenant en outre au moins un support, diluant et/ou excipient pharmaceutiquement acceptable.

13. Composition pharmaceutique pour son utilisation dans le traitement d'une maladie associée à une amyloïdose selon la revendication 11 ou 12, comprenant en outre un sel métallique monovalent ou divalent.

14. Composition pharmaceutique pour son utilisation dans le traitement d'une maladie associée à une amyloïdose selon la revendication 13, dans laquelle ledit métal est choisi dans le groupe constitué par le sodium, le magnésium, le calcium et le zinc.


### SEQUENCE LISTING

<110> Agency for Science, Technology and Research
<120> Anti-amyloidogenic, alpha-helix breaking ultra-small peptide therapeutics
<130> A32079WOEP
<150> SG 201104978-0
   <151> 2011-07-07
<160> 61
<170> PatentIn version 3.5
<210> 1
   <211> 7
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 1
   Leu Ile Val Ala Gly Asp Asp
   1               5
<210> 2
   <211> 7
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 2
   Leu Ile Val Ala Gly Glu Glu
   1 5
<210> 3
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 3
   Leu Ile Val Ala Gly Asp
   1 5
<210> 4
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 4
   Ile Leu Val Ala Gly Asp
   1 5
<210> 5
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 5
   Leu Ile Val Ala Ala Asp
   1 5
<210> 6
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 6
   Leu Ala Val Ala Gly Asp
   1               5
<210> 7
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 7
   Ala Ile Val Ala Gly Asp
   1               5
<210> 8
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 8
   Leu Ile Val Ala Gly Glu
   1               5
<210> 9
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 9
   Leu Ile Val Ala Gly Lys
   1               5
<210> 10
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 10
   Leu Ile Val Ala Gly Ser
   1               5
<210> 11
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 11
   Ile Leu Val Ala Gly Ser
   1               5
<210> 12
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 12
   Ala Ile Val Ala Gly Ser
   1 5
<210> 13
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 13
   Leu Ile Val Ala Gly Thr
   1 5
<210> 14
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 14
   Ala Ile Val Ala Gly Thr
   1 5
<210> 15
   <211> 5
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 15
   Leu Ile Val Ala Asp
   1               5
<210> 16
   <211> 5
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 16
   Leu Ile Val Gly Asp
   1               5
<210> 17
   <211> 4
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 17
   Ile Val Ala Asp
   1
<210> 18
   <211> 4
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 18
   Ile Ile Ile Asp
   1
<210> 19
   <211> 4
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 19
   Ile Ile Ile Lys
   1
<210> 20
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 20
   Ile Val Asp
   1
<210> 21
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 21
   Leu Pro Val Ala Gly Asp
   1               5
<210> 22
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 22
   Leu Ile Pro Ala Gly Asp
   1               5
<210> 23
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 23
   Leu Ile Val Pro Gly Asp
   1               5
<210> 24
   <211> 6
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 24
   Leu Ile Val Ala Pro Asp
   1               5
<210> 25
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 25
   Ile Pro Asp
   1
<210> 26
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 26
   Asn Pro Ile
   1
<210> 27
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 27
   Ile Pro Asn
   1
<210> 28
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 28
   Ile Pro Ile
   1
<210> 29
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 29
   Ala Pro Ala
   1
<210> 30
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 30
   Leu Pro Ile
   1
<210> 31
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 31
   Ile Pro Leu
   1
<210> 32
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 32
   Leu Pro Leu
   1
<210> 33
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 33
   Ala Pro Phe
   1
<210> 34
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<220>
   <221> MOD_RES
   <222> (3)..(3)
   <223> AMIDATION
<400> 34
   Lys Pro Ala
   1
<210> 35
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 35
   Leu Pro Asp
   1
<210> 36
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 36
   Leu Pro Glu
   1
<210> 37
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<220>
   <221> MOD_RES
   <222> (3)..(3)
   <223> AMIDATION
<400> 37
   Ile Pro Lys
   1
<210> 38
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 38
   Ala Pro Asp
   1
<210> 39
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 39
   Ile Pro Phe
   1
<210> 40
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 40
   Ile Pro Ser
   1
<210> 41
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 41
   Ile Pro Trp
   1
<210> 42
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 42
   Ala Pro Ser
   1
<210> 43
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<220>
   <221> MOD_RES
   <222> (3)..(3)
   <223> AMIDATION
<400> 43
   Asn Pro Lys
   1
<210> 44
   <211> 3
   <212> PRT
   <213> Artificial Sequence
<220>
   <223> Synthetic Peptide
<220>
   <221> MOD_RES
   <222> (1)..(1)
   <223> BLOCKED (N-terminal Protecting Group)
<400> 44
   Leu Pro Gly
   1
<210> 45
   <211> 6
   <212> PRT
   <213> Artificial Sequence
<220>
   <223> Synthetic Peptide
<220>
   <221> MOD_RES
   <222> (1)..(1)
   <223> ACETYLATION
<400> 45
   Leu Ile Pro Ala Gly Asp
   1               5
<210> 46
   <211> 6
   <212> PRT
   <213> Artificial Sequence
<220>
   <223> Synthetic Peptide
<220>
   <221> MOD_RES
   <222> (1)..(1)
   <223> ACETYLATION
<400> 46
   Leu Pro Val Ala Gly Asp
   1               5
<210> 47
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 47
   Leu Pro Glu
   1
<210> 48
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 48
   Leu Pro Asp
   1
<210> 49
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 49
   Leu Pro Gly
   1
<210> 50
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 50
   Leu Pro Leu
   1
<210> 51
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 51
   Ile Pro Ile
   1
<210> 52
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 52
   Ile Pro Asp
   1
<210> 53
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 53
   Ile Pro Ser
   1
<210> 54
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 54
   Ile Pro Trp
   1
<210> 55
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 55
   Ile Pro Phe
   1
<210> 56
   <211> 3
   <212> PRT
   <213> Artificial Sequence
<220>
   <223> Synthetic Peptide
<220>
   <221> MOD_RES
   <222> (1)..(1)
   <223> ACETYLATION
<220>
   <221> MOD_RES
   <222> (3)..(3)
   <223> AMIDATION
<400> 56
   Ile Pro Lys
   1
<210> 57
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 57
   Ala Pro Phe
   1
<210> 58
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 58
   Ala Pro Asp
   1
<210> 59
   <211> 3
   <212> PRT
   <213> Artificial Sequence
<220>
   <223> Synthetic Peptide
<220>
   <221> MOD_RES
   <222> (1)..(1)
   <223> ACETYLATION
<400> 59
   Ala Pro Ser
   1
<210> 60
   <211> 3
   <212> PRT
   <213> Artificial Sequence
<220>
   <223> Synthetic Peptide
<220>
   <221> MOD_RES
   <222> (1)..(1)
   <223> ACETYLATION
<220>
   <221> MOD_RES
   <222> (3)..(3)
   <223> AMIDATION
<400> 60
   Asn Pro Lys
   1
<210> 61
   <211> 6
   <212> PRT
   <213> Artificial Sequence
<220>
   <223> Synthetic Peptide
<220>
   <221> MOD_RES
   <222> (1)..(1)
   <223> ACETYLATION
<400> 61
   Asn Phe Gly Ala Ile Leu
   1               5